# EUROPEAN PATENT APPLICATION

(11) **EP 1 721 568 A1**
(43) Date of publication of application: **15.11.2006**
(21) Application number: 05719642.0
(22) Date of filing: 28.02.2005
(51) Int. Cl.: A61B 1/06

(54) **ENDOSCOPE**

(30) Priority: 02.03.2004 JP 2004057914; 18.11.2004 JP 2004334882
(71) Applicant: Olympus Corporation, Tokyo 151-0072 (JP)
(72) Inventor: Tsuji, Kiyoshi, Tokyo 192-8512 (JP); HAGIHARA, M. Olympus Int. Prop. Services Co. Ltd., Hachioji-shi, Tokyo 192-8512 (JP)
(74) Representative: von Hellfeld, Axel
(86) International application number: PCT/JP2005/003325
(87) International publication number: WO 2005/082230

(57) **Abstract**

An endoscope includes a slender insertion section of which the distal portion incorporates an image capturing device, and the back end of this insertion section is provided with an operating section to be gripped. A cable section is provided wherein a light guide cable for transmitting illumination light, and a signal cable connected to the image capturing device are integrally or separately extended from the operating section, and the connector of the edge portion of the cable section is freely detachably mounted to each of a light source device and a signal processing device. A mounting/detaching portion for freely detachably connecting a first unit serving as an insertion section side and a second unit serving as a cable section side is provided around between the distal end of the operating section and the proximal end thereof.

## Description

### Technical Field

The present invention relates to an endoscope for subjecting a body cavity or the like to endoscopy.

### Background Art

In recent years, endoscopes capable of subjecting the inside of a test object to inspection in the medical-application and industrial-application fields have been widely employed.

As for a conventional example, there is a rigid endoscope including a hard insertion section, which incorporates an image capturing device. With this rigid endoscope, a lig ht guide cable and a scope cable are integrated within the rigid endoscope main body.

Accordingly, in the event of changing the line of sight between that of a direct viewing type and a perspective type during surgery, a light guide connector at the terminal portion of a light guide cable and a connector for a signal processing device (processor device), which are in unclean areas in general, need to be detached and mounted again; but a surgeon in a clean area cannot perform the process thereof directly, consequently resulting in deterioration in workability thereof.

Conversely, for example, as for a first preceding example, Japanese Unexamined Patent Application Publication No. 2003-190081 has disclosed a rigid endoscope in which a rigid endoscope main body includes a hard insertion section which incorporates an image capturing device at the distal portion thereof, and a cable section which can be freely detached and mounted at an operating section in the rigid endoscope main body are separately provided.

With this first preceding example, wireless signal transmission/reception means is provided in the rigid endoscope main body, which is configured so as to transmit a signal image-captured to an external signal processing device wirelessly.

Also, with Japanese Unexamined Patent Application Publication No. 2002-369789 as a second preceding example, a rigid endoscope is configured wherein a light guide cable can be detached and mounted to an endoscope main body which incorporates an image capturing device, and also a light emitting element, wherein a signal image-captured by the image capturing device is emitted by the light emitting element, the light is transmitted by a light guide within the light guide cable, and is received by a light receiving element disposed within a light source device to convert this into an electric signal, and this signal is transmitted to a picture signal processing circuit.

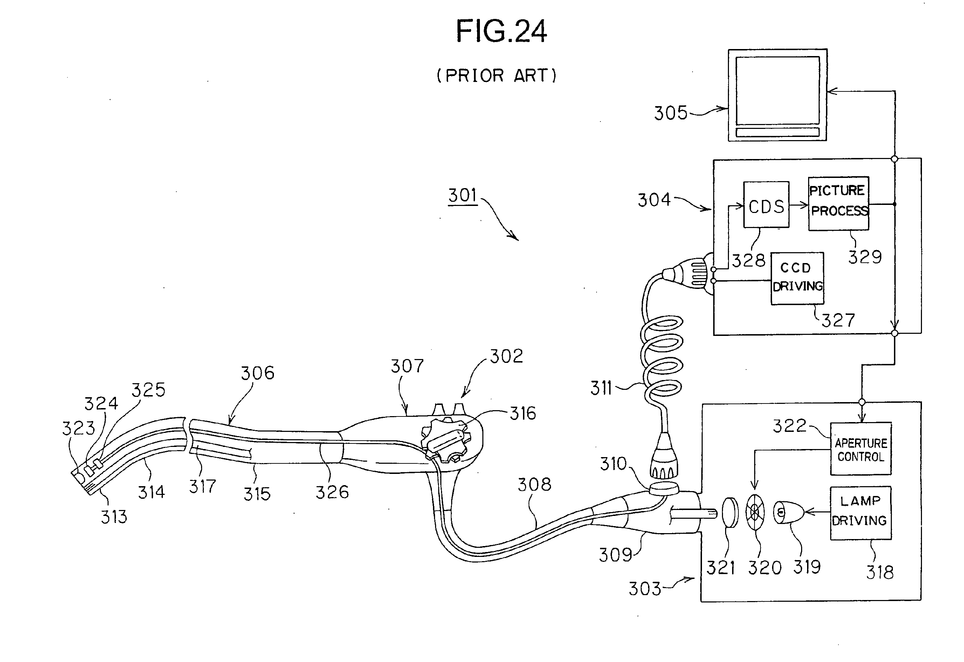

Also, an endoscope 302 as a third preceding example, which constitutes an endoscope device 301, such as illustrated in Fig. 24, exists. This endoscope device 301 includes the endoscope 302, a light source device 303 which supplies illumination light to this endoscope 302, a processor device 304 for subjecting an image capturing device built in the endoscope 302 to signal processing, and a monitor 305 for displaying an image captured by the image capturing device in response to the input of a picture signal to be output from the processor device 304.

The endoscope 302 includes an insertion section 306 having flexibility, an operating section 307 provided at the back end of this insertion section 306, and a universal cable 308 extended from this operating section 307. A terminal connector 309 of this universal cable 308 is connected to the light source device 303.

Also, an electric cable 311, which is connected to an electric connector 310 provided at the side surface of this connector 309, is connected to the processor device 304 for performing signal processing. The picture signal generated by the processor device 304 is output to the monitor 305.

The insertion section 306 of the endoscope 302 comprises a hard distal portion 313 provided at the distal end thereof, a bending portion 314 which can be bent, and a flexible portion 315 having flexibility. The bending portion 314 can be bent by performing an operation for moving a bending knob 316 provided in the operating section 307 rotationally.

Also, a light guide 317 for transmitting illumination light is inserted into the insertion section 306, this light guide 317 passes through the operating section 307, and further the universal cable 308, and the end thereof is mounted on the light source device 303 as a light guide base extruding from the end face of the connector 309.

Subsequently, the illumination light of a lamp 319 which is turned on by the lamp driving electric power from a lamp driving circuit 318 within the light source device 303 passes through an aperture 320, and further is condensed at a condenser lens 321, and is made incident into the end face of the light guide base.

This illumination light incident into the end face of the light guide base is transmitted by the light guide 317, and is emitted from the distal end face of the light guide 317 fixed to the illumination window of the tip portion 313, thereby illuminating a subject such as an affected portion or the like.

As for the illuminated subject, an objective lens 323 attached to an observation window adjacent to the illumination window forms the optical image of the subject at the image-forming position thereof. Acharge-coupled device (abbreviated as CCD) 324 is disposed at this image-forming position.

This CCD 324 is subjected to current amplification by a buffer IC 325, following which is connected to the electric connector 310 via a signal cable 326 inserted into the insertion section 306 or the like, and from this electric connector 310 further is connected to a CCD driving circuit 327 and a CDS circuit 328 within the processor device 304 via the electric cable 311.

Subsequently, the optical image formed on the image capturing surface of the CCD 324 is subjected to photoelectric conversion by applying the CCD driving signal from this CCD driving circuit 327 to the CCD 324, and is output as a CCD output signal.

This CCD output signal is input to the CDS circuit 328, the signal components thereof are extracted to be converted into a base band signal, and is input to a picture process circuit 329, and is converted into a picture signal to be output to the monitor 305, and the subject image captured by the CCD 324 is displayed on the display screen of the monitor 305 as an endoscope image.

Also, the picture signal to be output from the picture process circuit 329 is input to an aperture control circuit 322 within the light source device 303, this aperture control circuit 322 generates a light adjustment signal from this picture signal, and controls the opening amount of the aperture 320 by this light adjustment signal to perform light adjustment so as to obtain a suitable amount of illumination light.

With the above first preceding example, transmission of a signal between the image ' capturing device and the signal processing device is preformed wirelessly, and consequently, in the event of the image signal captured by the image capturing device is transmitted to the signal processing device wirelessly for example, deterioration in image quality readily occurs by surrounding noise being mixed in the signal to be received at the signal processing device, or the like.

Also, a signal for driving the image capturing device is transmitted wirelessly, so that the signal sometimes becomes a factor for necessity of countermeasures to noise as to surrounding electrical machinery and apparatuses.

Also, the second preceding example employs a configuration wherein light is transmitted using a part of the light guide within the light guide cable, which complicates the configuration of the connection portion at particularly the light receiving element side, leading to a drawback in that manufacturing costs increase.

Also, the preceding example illustrated in Fig. 24 has the same drawback in the case of a rigid endoscope main body wherein the light guide cable and the scope cable are integrated.

It is convenient to allow a surgeon to readily replace and use a part of the insertion section side of the endoscope during endoscopy.

With the above first preceding example, the light guide can be freely detached and mounted in the vicinity of the operating section in the electronic endoscope which incorporates the image capturing device at the distal portion of the insertion section, but transmission of a signal is performed wirelessly, and consequently, the signal is readily affected by noise.

Also, the second preceding example employs a configuration wherein the signal captured by the image capturing device is transmitted using a light signal in the electronic endoscope which incorporates the image capturing device at the distal portion of the insertion section, and accordingly cannot use conventional signal processing devices.

The present invention has been made in the light of the above points, and it is an object of the present invention to provide an endoscope which can retain compatibility wherein conventional light source devices and signal processing devices can be used, and allow a surgeon to readily replace the previously used endoscope by another endoscope even during endoscopy or the like.

### Disclosure of Invention

### Means for Solving the Problems

The present invention provides an endoscope comprising: a slender insertion section which incorporates image capturing means at the distal portion; an operating section, which is provided at the rear end of the insertion section, to be gripped; and a cable section in which a light guide cable for transmitting illumination light from the operating section and a signal cable connected to the image capturing means are extended integrally or separately; wherein the connector of the end face of the cable section can be freely detachably mounted to each of a light source device and a signal processing device;
and wherein a mounting/detaching portion is provided between the front end and the back end of the operating section for freely detachably connecting a first unit serving as the insertion section side and a second unit serving as the cable section side.

According to the above configuration, conventional light source devices and signal processing devices can be used, and also a surgeon can remove only the first unit serving as the insertion section side, and can readily replace this unit by another first unit which has a different type of image capturing means and the like even during endoscopy.

### Brief Description of the Drawings

Fig. 1 is a block diagram illustrating the configuration of a medical endoscope system including a first embodiment of the present invention.
Fig. 2 is a block diagram illustrating the configuration of a medical endoscope system including a second embodiment of the present invention.
Fig. 3 is a diagram illustrating the configuration near a cable unit according to a third embodiment.
Fig. 4 is a diagram illustrating the configuration near a cable unit of a rigid endoscope according to a modification.
Fig. 5 is a block diagram illustrating an endoscope system including a fourth embodiment.
Fig. 6 is a block diagram illustrating an endoscope system according to a first modification.
Fig. 7A is a block diagram illustrating an endoscope system according to a second modification.
Fig. 7B is a diagram illustrating the internal configuration of an integral connector according to the second modification.
Fig. 8 is a block diagram illustrating an endoscope system according to a third modification.
Fig. 9 is a configuration diagram illustrating an endoscope system including a fifth embodiment of the present invention.
Fig. 10 is a diagram illustrating the configuration of an endoscope according to the fifth embodiment in which a mounting/detaching portion is provided.
Fig. 11 is a schematic view illustrating the configuration on the periphery of the mounting/detaching portion in a perspective manner.
Fig. 12A is a schematic view illustrating the configuration of a mounting/detaching portion according to a first modification in a perspective manner.
Fig. 12B is a schematic view illustrating the configuration of a mounting/detaching portion having a somewhat different configuration from Fig. 12A in a perspective manner.
Fig. 13 is a sectional view illustrating the configuration of a mounting/detaching portion according to a second modification.
Fig. 14 is a schematic view illustrating the configuration of a mounting/detaching portion according to a third modification in a perspective manner.
Fig. 15 is a sectional view illustrating the configuration on the periphery of a coil in the mounting/detaching portion in a mounting state.
Fig. 16 is a schematic view illustrating the configuration of a mounting/detaching portion according to a fourth modification in a perspective manner.
Fig. 17 is an explanatory diagram illustrating the configuration of a transmission/reception portion using light.
Fig. 18 is a configuration diagram illustrating an endoscope system including a sixth embodiment of the present invention.
Fig. 19 is a schematic view illustrating the configuration on the periphery of a mounting/detaching portion in a perspective manner.
Fig. 20 is a schematic view illustrating the configuration of a mounting/detaching portion according to a first modification in a perspective manner.
Fig. 21 is a schematic view illustrating the configuration of a mounting/detaching portion according to a second modification in a perspective manner.
Fig. 22 is a diagram illustrating the principal portions of the configuration of the mounting/detaching portion according to the second modification.
Fig. 23 is a diagram illustrating the configuration of an endoscope according to a third modification.
Fig. 24 is a configuration diagram illustrating an endoscope system including an endoscope of a preceding example.

### Best Mode for Carrying Out the Invention

Preferred embodiments of the present invention will be described below with reference to the drawings.

### (First Embodiment)

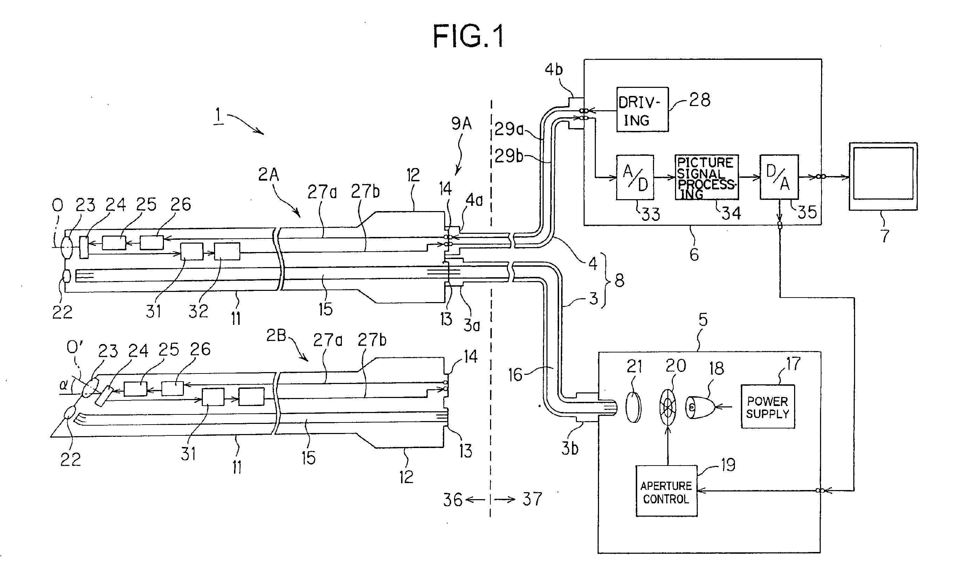

Fig. 1 illustrates the configuration of a medical endoscope system including a first embodiment of the present invention. As illustrated in Fig. 1, this endoscope system 1 comprises a direct-viewing-type rigid endoscope main body 2A, a perspective-type rigid endoscope main body 2B, a light guide cable section 3 and a signal cable section 4 each of which one end is freely detachably connected to the rigid endoscope main body 2I (I is A or B), a light source device 5 to which the other end of the light guide cable section 3 is freely detachably connected for supplying illumination light, a processor device 6 to which the other end of the signal cable section 4 is freely detachably connected for performing signal processing, and a monitor 7 to which the processor device 6 is freely detachably connected for displaying the corresponding image in response to a picture signal being input.

Connecting the light guide cable section 3 and the signal cable section 4, which constitute a cable unit 8 that can be freely detachably mounted to the rigid endoscope main body 2I, enables the light source device 5 and the processor device 6 to be freely detachably connected, thereby forming a medical rigid endoscope 9I for performing endoscopy.

The rigid endoscope 9I according to the present embodiment is configured so as to have two units of the rigid endoscope main body 2I serving as a front side endoscope portion (first unit), and a rear side endoscope portion (second unit) which can be freely detachably mounted at a mounting/detaching portion near the proximal end of an operating section 12 of the front side endoscope portion. Note that the direct -viewing-type rigid endoscope main body 2A and the perspective-type rigid endoscope main body 2B differ on only an optical system portion which illuminates and captures an image at the distal portion of an insertion section 11, as described below.

The endoscope main body 2I includes the hard insertion section 11 made up of slender cylindrical stainless steal or the like, and the operating section (also referred to as gripper) 12 to be gripped by a surgeon or the like, which is disposed at the proximal end of the insertion section 11. With the operating section 12, for example, the proximal end face is provided with a light guide connector 13 and a signal connector 14, which are freely detachably connected to a connector receptacle 3a provided at one end of the light guide cable section 3, and a signal connector receptacle 4a provided at one end of the signal cable section 4 respectively. That is to say, the endoscope main body 2I has connection portions to which the light guide cable section 3 and the signal cable section 4 are freely detachably connected.

Also, a light guide connector 3b provided at the other end of the light guide cable section 3, and a signal connector 4b provided at the other end of the signal cable section 4 are freely detachably connected to the light source device 5 and the processor device 6 respectively.

A light guide 15 for transmitting illumination light is inserted into the insertion section 11, and the proximal end of this light guide 15 reaches the light guide connector 13 serving as an incident end of illumination light. As illustrated in Fig. 1, the illumination light from the light source device 5 is supplied to the light guide 15 via a light guide 16 within the light guide cable section 3 by connecting the light guide cable section 3 to the light guide connector 13. Note that for example, the light guide connector 13 will be abbreviated to connector 13, and the signal connector 14 abbreviated to connector 14.

The light source device 5 incorporates a light source lamp 18 which is lit by electric power from a power supply circuit 17, the light of the light source lamp 18 is adjusted to a suitable amount of light by passing through an aperture 20 of which the passage amount of light is controlled by an aperture control circuit 19, following which is condensed by a condenser lens 21 to be supplied to the light guide 16 of the light guide cable section 3, and further supplied to the light guide 15 of the endoscope main body 2I via the light guide 16.

The distal end serving as the emitting end of the light guide 15 is fixed within the distal portion of the insertion section 11, the illumination light transmitted to the distal end face of the light guide 15 is emitted to the forward the illumination lens 22 via an illumination lens 22 attached to an illumination window, and the insertion section 11 illuminates a subject such as an affected portion to be inspected or treated within a body, such as the abdomen or the like, to be injected by a trocar or the like.

The distal portion of the insertion section 11 is provided with an observation window (image capturing window) adjacent to the illumination window, the observation window is attached with an objective lens 23, the image-forming position of the objective lens 23 is disposed with a charge-coupling device (abbreviated as CCD) 24 serving as a solid image capturing device for example, and an optical image formed on this image capturing surface is subjected to photoelectric conversion.

As illustrated in Fig. 1, with the direct-viewing-type rigid endoscope main body 2A, the optical axis O of the objective lens 23 is the direction parallel to the axis of the insertion section 11, and a forward direction parallel to the axis of the insertion section 11 is the line of sight by means of the objective lens 23. Note that the illumination light to be emitted from the distal end face of the light guide 15 via the illumination lens 22 illuminates the forward side parallel to the axis of the insertion section 11 so as to illuminate the line of sight of the objective lens 23.

On the other hand, with the perspective-type rigid endoscope main body 2B, the distal end face of the insertion section 11 is formed so as to direct in an oblique direction shifted from the direction orthogonal to the axial direction of the insertion section 11, the illumination window and observation window are adjacently provided on the distal end face in this oblique direction, and the corresponding illumination lens 22 and objective lens 23 are attached thereto respectively. Also, the distal end of the light guide 15 of which around the tip is bent is fixed to the inside of the illumination lens 22. Also, the CCD 24 is disposed at the image-forming position of the objective lens 23. The optical axis O' of the objective lens 23 takes the perspective direction making an angle α for example with the axial direction of the insertion section 11 as line of sight.

Even with either of the rigid endoscope main bodies 2I, the CCD 24 within the distal portion is connected to the contact point of the signal connector 14 by a signal line 27a inserted into the insertion section 11 and the operating section 12 via a waveform shaping circuit 25 and a buffer circuit 26 which are built in around the distal end portion for example.

As illustrated in Fig. 1, the CCD driving signal from a driving circuit 28 within the processor device 6 is applied to the CCD 24 via a signal line 29a within the signal cable section 4 and the signal line 27a within the rigid endoscope main body 2I by being connected to the processor device 6 via the signal cable section 4 connected to the signal connector 14 of the rigid endoscope main body 2I.

The CCD 24 outputs signal charge subjected to photoelectric conversion by application of this CCD driving signal as a CCD output signal. From this CCD output signal, signal components are extracted by the CDS circuit 31 disposed near the CCD 24, and further are subjected to current amplification by the buffer circuit 32.

The signal subjected to current amplification by the buffer circuit 32 is input to the processor device 6 via the signal line 27b inserted into the insertion section 11 and the operating section 12, and the signal line 29b within the signal cable section 4. The signal input to the processor device 6 is converted into a digital signal by an A/D conversion circuit 33, following which is subjected to signal processing by a picture signal processing circuit 34 to generate a digital picture signal.

This digital picture signal is input to a D/A conversion circuit 35, and is converted into an analog picture signal to be output to the monitor 7 from the output end thereof, and also is output to the aperture control circuit 19 of the light source device 5. The aperture control circuit 19 integrates the luminance level of the input picture signal with a predetermined cycle, or generates a light adjustment signal (luminance control signal) by passing it through a lowpass filter, compares this luminance level with the reference luminance level, and adjusts the opening amount of the aperture 20 by the difference signal from the reference level, whereby the amount of illumination light can be adjusted so as to obtain a luminance appropriate for observation of an image to be displayed on the monitor 7.

With the endoscope system 1 thus configured, the direct-viewing-type rigid endoscope 9A comprises the light guide cable section 3 and the signal cable section 4, which can be freely detachably mounted to the rigid endoscope main body 2A. Also, the perspective-type rigid endoscope 9B comprises the light guide cable section 3 and the signal cable section 4, which can be freely detachably mounted to the rigid endoscope main body 2B.

In this case, the rigid endoscope main bodies 2A and 2B are employed in the medical field, so, as illustrated in Fig. 1, the light guide cable section 3 and the signal cable section 4 in the vicinity of the operating section 12 are in a clean area 36 which is set in a clean state, whereby a surgeon who performs inspection and treatment in the clean area 36 can operate these without any restriction. However, the connectors 3b and 4b sides at the back end portion in the light guide cable section 3 and the signal cable section 4 are in an unclean area 37 which is not set in a clean state, so that a surgeon who exists in the clean area 36 cannot operate these during inspection and during surgery for all practical purposes.

With the present embodiment, each of the rigid endoscopes 9I is configured so as to be separated into the rigid endoscope main body 2I, and the light guide cable section 3 and the signal cable section 4 which can be freely detachably mounted at the operating section 12, whereby the rigid endoscope main body 2I can be readily replaced within the clean area 36, which is the features of the present embodiment.

Accordingly, in the event that the surgeon grips and uses the operating section 12 in a direct-viewing or perspective observation field of view state, and wants to change a field of view during surgery, the surgeon can readily remove the connectors 13 and 14 portions at the operating section 12 in the clean area 36, and replace the endoscope by a rigid endoscope main body to be employed for changing a field of view. That is to say, a freely detachably mounting connection mechanism which facilitates replacement within the clean area 36 is formed, whereby the endoscope can be readily detached and replaced with another endoscope.

Also, as described above, with the present embodiment, each of the rigid endoscopes 9I is configured so as to be separated into the rigid endoscope main body 2I, and the cable unit 8 constituted of the light guide cable section 3 and the signal cable section 4 which can be freely detachably mounted thereupon, whereby each of the rigid endoscopes 9I can be readily subjected to sterilization or disinfection.

That is to say, in the event of attempting to subject each of the rigid endoscopes 9I to sterilization or disinfection, when the rigid endoscope main body 2I and the cable unit 8 cannot be separated, the long cable unit 8 also needs to be subjected to sterilization or disinfection, which makes a process such as storing or the like troublesome, but with the present embodiment, the rigid endoscope main body 2I and the cable unit 8 can be separated, thereby facilitating the process.

Also, the rigid endoscope main body 2I and the cable unit 8 sometimes have different tolerance as to sterilization and disinfection due to a material to be used, and in this case, it is necessary to perform sterilization and disinfection in light of lower tolerance, but with the present embodiment, the rigid endoscope main body 2I and the cable unit 8 can be separated, thereby enabling sterilization and disinfection to be performed in light of each of tolerances. Also, even in the event of performing mending or the like, with the present embodiment wherein the rigid endoscope main body 2I and the cable unit 8 can be separated, mending or the like can be performed economically. Also, the present embodiment can be realized with a simple configuration.

Also, according to the rigid endoscope 9I of the present embodiment, a conventional light source device and a conventional signal processing device (processor device), which can be used when the rigid endoscope main body 2I and the cable unit 8 are integrated, can be used without any modification.

Accordingly, the rigid endoscope 9I according to the present embodiment can be realized with a simple configuration, and has wide versatility, thereby providing a user-friendly endoscope. In addition, this can be manufactured at low cost.

Also, the above description has been made wherein the rigid endoscope 9I comprises the rigid endoscope main body 2I and the cable unit 8, but the rigid endoscope main body 2I can be regarded as a rigid endoscope.

In this case, the rigid endoscope itself can be readily replaced with another endoscope having a different field of view in the clean area 36. Also, in the case of regarding the rigid endoscope main body 2I as a rigid endoscope, the present embodiment can be applied to a case in which the connectors 3b or 4b side of the cable unit 8 are configured integral with the light source device 5 or the processor device 6.

Accordingly, even with the following embodiments, description will be made wherein the rigid endoscope 9I or the like comprises the rigid endoscope main body 2I, and the cable unit 8 freely detachably mounted thereupon, as with the first embodiment, but the rigid endoscope main body 2I can be regarded as a rigid endoscope.

### (Second Embodiment)

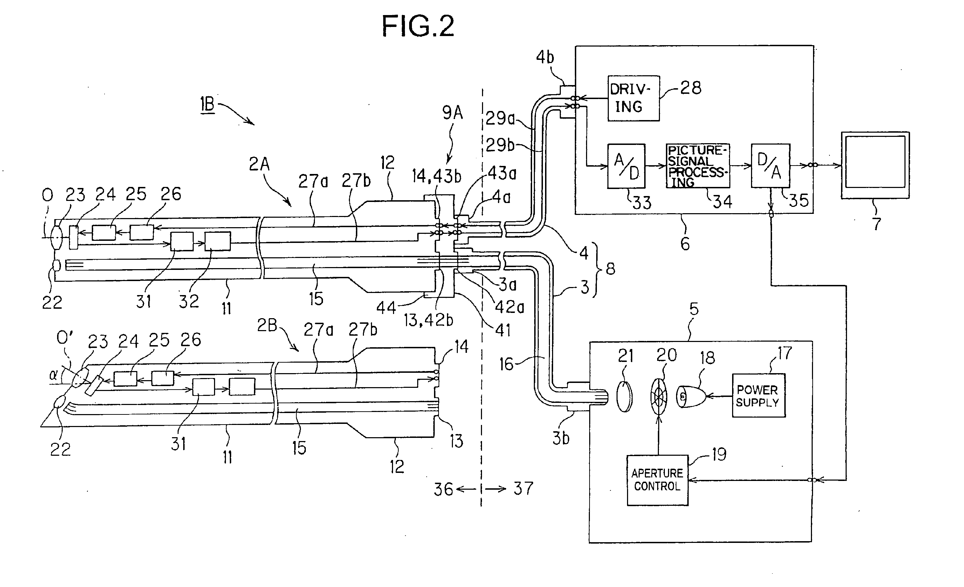

Next, a second embodiment of the present invention will be described with reference to Fig. 2. Fig. 2 illustrates an endoscope system 1B including the second embodiment.

This endoscope system 1B enables connection between the rigid endoscope main body 2I, and the light guide cable section 3 and the signal cable section 4 to be performed via a connection adapter 41 in the endoscope system 1 in Fig. 1.

That is to say, connectors 42a and 43a to be freely detachably connected with the connector receptacles 3a and 4a of the light guide cable section 3 and the signal cable section 4 are provided at one end portion side of the connection adapter 41. Also, connector receptacles 42b and 43b, which can be freely detachably connected to the connectors 13 and 14 in the rigid endoscope main body 2I by one-touch operation, are provided at the other end portion side of the connection adapter 41. The other configurations are the same as those in the first embodiment.

This connection adapter 41 has a configuration wherein a mounting portion 44 which substantially fits with the proximal end of the operating section 12 is provided for example, positioning is made between the proximal end of the operating section 12 and the circumferential direction, and the mounting portio n 44 is pressed against the operating section 12, whereby the connection adapter 41 can be freely detachably attached by one-touch operation to the operating section 12 riding over a small protrusion.

Accordingly, with the present embodiment, the connector receptacles 3a and 4a of the light guide cable section 3 and the signal cable section 4 are connected to the connectors 42a and 43a at one end portion side of the connection adapter 41 respectively, and the other end side of this connection adapter 41 is connected to the rigid endoscope main body 2A or 2B, thereby performing endoscopy or the like.

In the event of performing endoscopy by changing a field of view, the previously used endoscope can be readily replaced with a rigid endoscope main body 2J (J is other than I) which attempts to perform endoscopy with a changed field of view by disconnecting the rigid endoscope main body 21 from the connection adapter 41, whereby the rigid endoscope main body 2J can be used.

Thus, according to the present embodiment, the rigid endoscope main body 2I can be replaced with the rigid endoscope main body 2J in a simple manner as compared with the first embodiment, whereby the rigid endoscope main body 2J can be used. The present embodiment has the same other advantages as the first embodiment.

### (Third Embodiment)

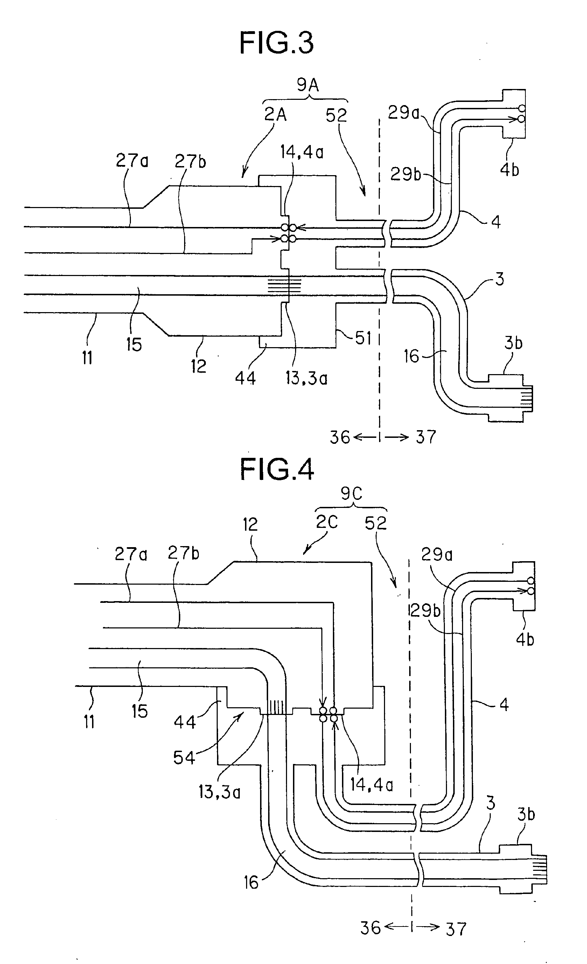

Next, a third embodiment of the present invention will be described with reference to Fig. 3. The present embodiment employs an integral connector (or integral adapter) 51 wherein the connector receptacles 3a and 4a which are the sides connected to the endoscope main bodies 2A or 2B in the light guide cable section 3 and the signal cable section 4 in the first embodiment. In other words, the present embodiment provides a configuration wherein with the second embodiment in Fig. 2, the connection adapter 41 is integrated with a part of the connector receptacles 3a and 4a sides in the light guide cable section 3 and the signal cable section 4.

That is to say, the rigid endoscope 9A(or 9B) according to the third embodiment comprises the rigid endoscope main body 2A, and a cable unit 52 wherein the integral connector 51, which can be freely detachably connected to the rigid endoscope main body 2A, is provided.

With the integral connector 51, a light guide connector receptacle 3a and a signal connector receptacle 4a which are connected to the light guide connector 13 and the signal connector 14 of the operating section 12 respectively are provided, and also the mounting portion 44 having recesses of the inside diameters which can be fitted with the outside diameters of the proximal ends of the operating section 12 is provided at the outer circumferential side.

The light guide cable section 3 and the signal cable section 4 are extended from this integral connector 51, and are freely detachably connected to the light source device 5 and the processor device 6 respectively. The other configurations are the same as those shown in the first embodiment or the second embodiment.

According to the present embodiment, the surgeon can readily freely detachably mount the cable unit 52 in the clean area 36 by detachable mount operations of the integral connector 51 as to the rigid endoscope main body 2A or 2B.

Accordingly, as illustrated in Fig. 3, the surgeon performs endoscopy or surgery under endoscopy using the rigid endoscope 9A in which the cable unit 52 is connected to the rigid endoscope main body 2A for example, and during the surgery, in the event of desiring to change line of sight from direct viewing to perspective viewing, the surgeon can readily replace the rigid endoscope 9A with the rigid endoscope 9B to use the rigid endoscope 9B as follows.

Specifically, the surgeon can replace the rigid endoscope 9A with a rigid endoscope 9B to use the rigid endoscope 9B by removing the integral connector 51 by performing an operation for separating the integral connector 51 from the operating section 12, and positioning between the mounting portion 44 of this integral connector 51 and the operating section 12 of the perspective-type rigid endoscope main body 2B, and mounting the integral connector 51 on the perspective-type rigid endoscope main body 2B by an operation for pressing the integral connector 51 against the perspective-type rigid endoscope main body 2B.

Fig. 4 illustrates the configuration in the vicinity of the cable section of a rigid endoscope 9C according to a modification. The rigid endoscope 9C comprises a rigid endoscope main body 2C, and the cable unit 52 including the integral connector 51 which can be freely detachably mounted on the rigid endoscope main body 2C.

An endoscope system including the present modification also includes the rigid endoscope 9C illustrated in Fig. 4 in addition to the rigid endoscope 9A and so forth illustrated in Fig. 3.

The rigid endoscope main body 2C differs from the direct-viewing-type rigid endoscope main body 2A for example in that the light guide connector 13 and the signal connector 14 provided on the proximal end face of the operating section 12 constitute a connector portion 54 which is provided on the side surface of the operating section 12 in the same shape.

In addition, the integral connector 51 of the cable unit 52 illustrated in Fig. 3 is configured so as to be freely detachably mounted (connected) to the connector portion 54 provided on the side face of the operating section 12.

The surgeon can select and use the rigid endoscope main body 2A of which the proximal end face is provided with the light guide connector 13 and the signal connector 14, or the rigid endoscope main body 2C of which the side face is provided with the light guide connector 13 and the signal connector 14 in light of actual ease of use. Also, the surgeon can select and use the rigid endoscope main body 2A or the rigid endoscope main body 2C depending on a portion to be subjected to surgery.

The present modification has the same advantages as those in the case of Fig. 3. Also, as for an endoscope system, the types of rigid endoscopes which can be actually employed increase, and thus operability can be further improved.

### (Fourth Embodiment)

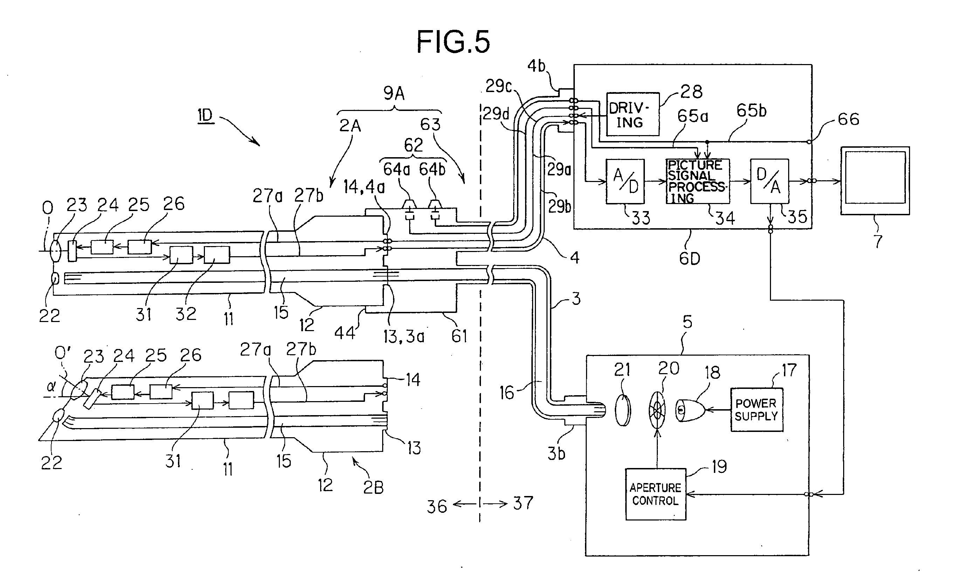

Next, a fourth embodiment of the present invention will be described with reference to Fig. 5. Fig. 5 illustrates an endoscope system 1D including the fourth embodiment.

The endoscope system 1D differs from the endoscope system 1 in Fig. 1 in that the connection sides as to the rigid endoscope main body 2I in the separate light guide cable section 3 and the separate signal cable section 4 are integrated into an integral connector (integral adapter) 61, and also a switch portion 62 for instructing and controlling a processor device 6D is provided in the integral connector 61. Accordingly, the rigid endoscope 9A according to the present embodiment comprises the rigid endoscope main body 2A, and a cable unit 63 in which the integral connector 61 to be freely detachably connected to the operating section 12 of the rigid endoscope main body 2A is provided.

The integral connector 61 is further provided with the switch portion 62 in the integral connector 51 illustrated in Fig. 3 for example. The switch portion 62 includes, for example, a freeze switch 64a and a release switch 64b. In Fig. 5, the two switches are illustrated, but one switch, or three or more than three switches may be provided.

The freeze switch 64a and the release switch 64b reach the contact points of a signal connector 4b via signal lines 29c and 29d within the signal cable section 4. Subsequently, these are freely detachably connected to the signal connector receptacles of the processor device 6D.

The processor device 6D differs from the processor device 6 in Fig. 1 in that signal lines 65a and 65b are further extended to the picture signal processing circuit 34 from the signal connector receptacles. In addition, the signal line 65b to be connected to the release switch 64b is further connected to a release instruction output end 66. Subsequently, the release instruction output end 66 is configured so as to transmit a release signal to an image recording device such as an unshown image filing device or the like to be connected to the control end thereof.

The other configurations are the same as those in the configuration illustrated in Fig. 1.

The present embodiment includes the advantages in the third embodiment, and also can perform operations such as a release instruction and so forth, whereby operability can be further improved.

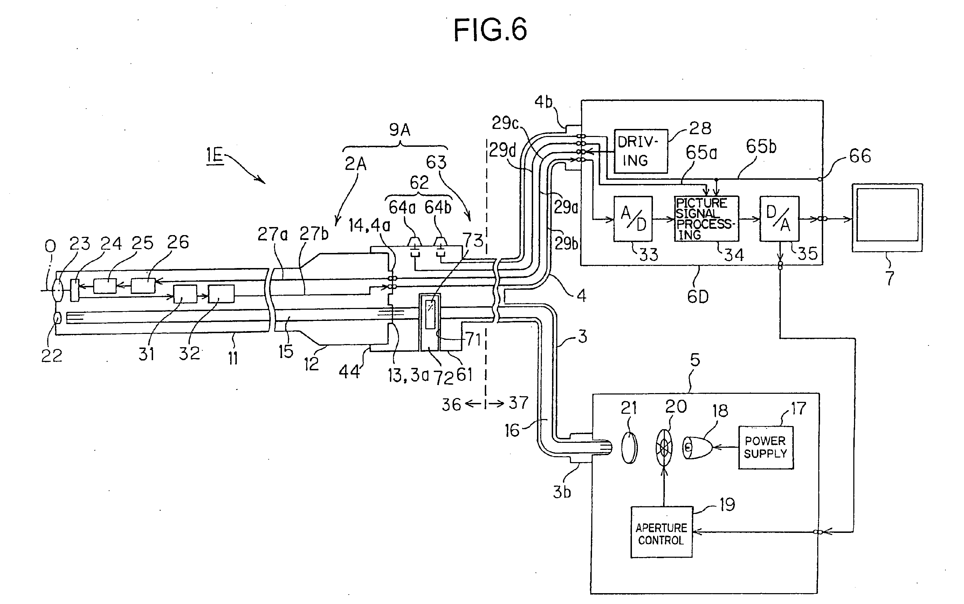

Fig. 6 illustrates an endoscope system 1E including a first modification. The endoscope system 1E differs from the endoscope system 1D in Fig. 5 in that a recess 71 is provided on the way to the light guide 16 within the integral connector 61, where a filter cassette 72 can be mounted. An optical filter 73 for restricting a wavelength band to be passed through is stored within the filter cassette 72.

The optical filter 73 is constituted of an infrared transmission filter for transmitting infrared light, an ultraviolet transmission filter for transmitting ultraviolet light, or the like, which is configured so as to perform special light observation (special light image capturing) under illumination such as infrared light, ultraviolet light, or the like by mounting the filter cassette 72 which differs as to the type of the built-in optical filter 73.

Note that in Fig. 6, the light guide cable section 3 and the signal cable section 4 are configured so as to be inserted into a single cable from the integral connector 61 to the backward side, and are branched into two at the proximal end side.

The other configurations are the same as those in Fig. 5.

The present modification includes the same advantages of the third embodiment and so forth, and also can obtain observation images using infrared light, ultraviolet light, or the like in addition to ordinary observation images using an ordinary visible region.

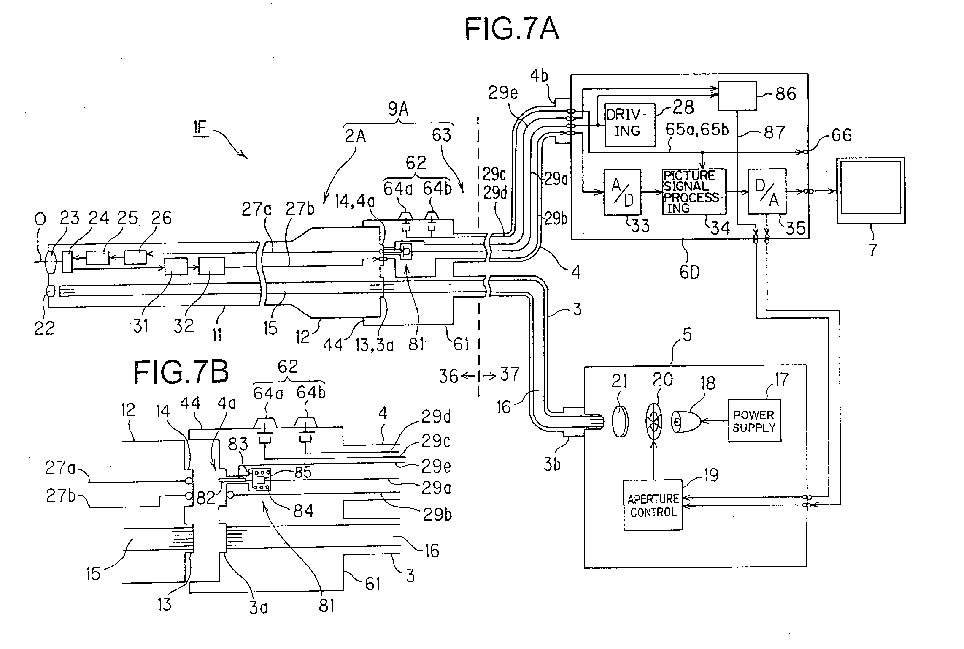

Fig. 7A illustrates the principal portions in an endoscope system 1F including a second modification. The endoscope system 1F differs from the endoscope system 1D in Fig. 5 in that a connection detecting unit 81 which detects whether or not the integral connector 61 is connected to the rigid endoscope main body 2A is provided within the integral connector 61, and control is performed for reducing illumination light to be supplied to the light guide 16 from the light source device 5 based on the connection detection result by the connection detecting unit 81.

As illustrated in Fig. 7B, a contact pin 82 constituting the connection detecting unit 81 for performing detection of connection regarding whether or not to connect to the contact point of the end portion of the signal line 27a is stored in the vicinity of the signal connector receptacle 4a within the integral connector 61 in a state pressed in the protruding direction by a coil-shaped spring 84 so as to slide into a pin hole. In this case, the spring 84 presses a contact piece 83 connected to the proximal end of the contact pin 82.

A contact piece 85, which is turned on/off, is disposed, for example, at the insi de of the spring 84, facing the contact piece 83. The contact piece 85 is connected to the signal line 29a, and the contact piece 83 facing the contact piece 85 is also connected to the signal line 29e.

The signal lines 29a and 29e are connected to an ON/OFF detection circuit 86 within the processor device 6E via the contact point of the signal connector 4b, and the ON/OFF detection circuit 86 transmits a connection detection signal to the aperture control circuit 19 of the light source device 5 via a signal line 87.

In the event that the connection detection signal, which has been turned on, is input, the aperture control circuit 19 performs ordinary operation, and in the event that the nonconnection detection signal, which has been turned off, is input, the aperture control circuit 19 narrows the aperture 20 down to the maximum, and sufficiently reduces illumination light to be supplied to the light guide 16 side.

With such a configuration, an arrangement may be made wherein only in the event that the integral connector 61 is actually connected to the rigid endoscope main body 2I, illumination light is emitted from the distal end face serving as the connector receptacle 3a of the light guide 16, and in the event that the integral connector 61 is not connected to the rigid endoscope main body 2I, illumination light is not emitted, or illumination light which is sufficiently reduced is emitted. Accordingly, illumination light in the case of not being used can be prevented from being emitted uselessly, and also near the distal end face of the light guide 16 can be prevented from heating.

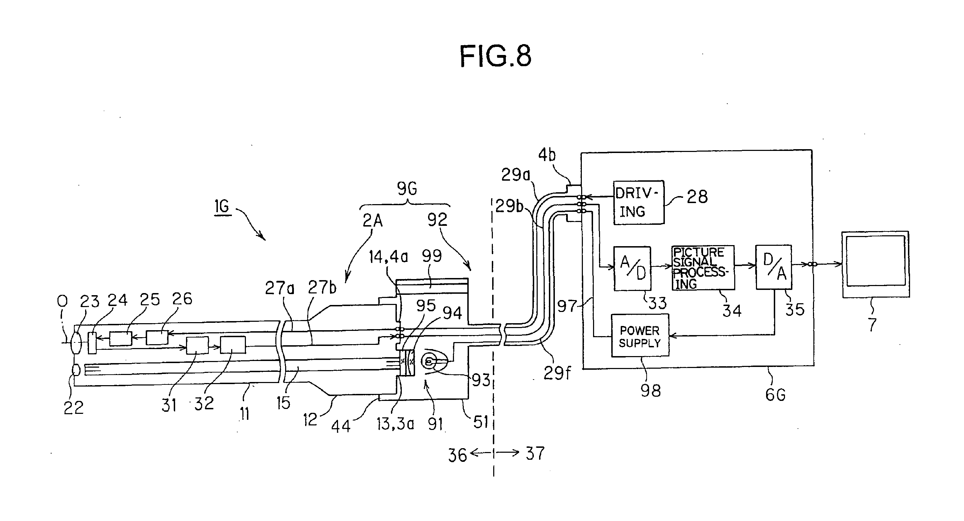

Fig. 8 illustrates an endoscope system 1G according to a third modification. The endoscope system 1G differs from the endoscope system including the rigid endoscope 9A having the integral connector 51 illustrated in Fig. 3 in that a light source unit 91 is provided within the integral connector 51, and a rigid endoscope 9G is constituted of a cable unit 92 not including the light guide cable section 3.

The light source unit 91 includes a light source lamp 93, a condenser lens 94 for condensing the illumination light of the light source lamp 93, and a cover glass 95 for covering a window facing the condenser lens 94, and is configured so as to supply the illumination light emitted at the light source lamp 93 to the light guide 15 by connecting the connector receptacle 3a, to which the cover glass 95 is attached, to the light guide connector 13.

Also, the integral connector 51 incorporates the light source unit 91, so only the signal cable section 4 is extended from the integral connector 51, and the signal connector 4b of the end portion of the signal cable section 4 is freely detachably connected to the processor device 6G

The processor device 6G differs from the processor device 6 in Fig. 1 for example in that the processor device 6G incorporates a power supply circuit 98 which is connected to the light source lamp 93 via the signal line 29f of which one end is connected to the light source lamp 93, and a signal line 97 which is connected to the signal line 29f. Also, the output signal from the D/Aconversion circuit 35 is input to the power supply circuit 98, and the amount of emission of the light source lamp 93 can be controlled using the output signal.

Also, a guide hole 99 for inserting a treatment tool is provided in this integral connector 51, whereby treatment may be performed by inserting a treatment tool.

Note that in the event that a channel for inserting a treatment tool is provided within the rigid endoscope main body 2A, the guide hole 99 is provided at the position facing the channel.

According to the present modification, the number of cables, which are connected to the rigid endoscope main body 2A, to be extended to the backward side and the like is reduced, whereby operability can be improved.

As described above, as for an instance using a rigid endoscope in surgery, description has been made based on needs for exchanging a direct-viewing-type endoscope and a perspective-type endoscope during surgery (during endoscopy), but further, there are needs for exchanging a rigid-endoscope-type endoscope and a flexible-endoscope-type endoscope, and accordingly, the endoscope according to the present embodiment is not restricted to a rigid endoscope, and can be applied to a flexible endoscope. Next, an embodiment of a flexible endoscope wherein the insertion section is flexible will be described.

### (Fifth Embodiment)

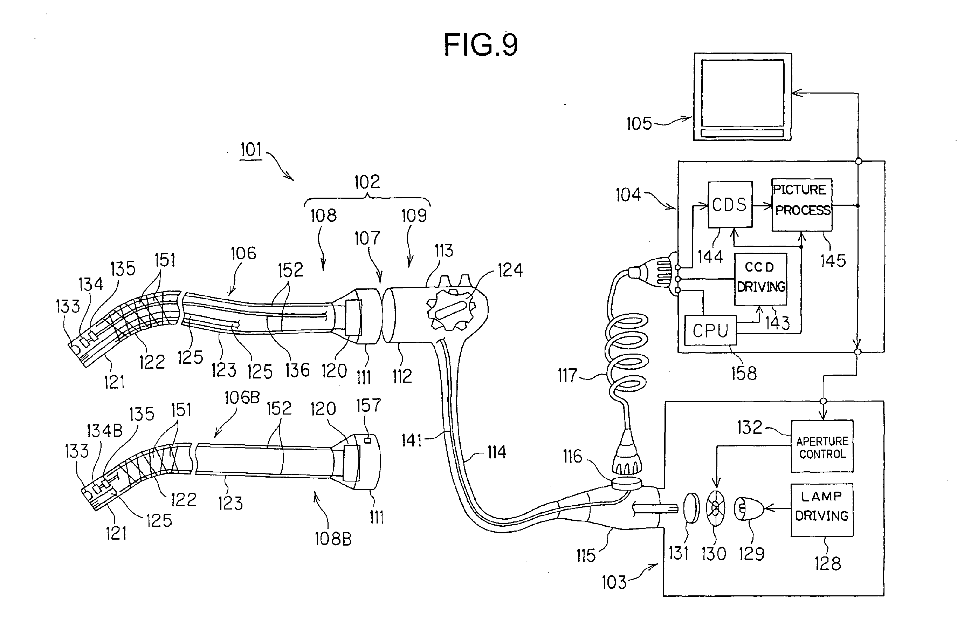

Next, a fifth embodiment of the present invention will be described with reference to Fig. 9 through Fig. 17. It is an object of the present embodiment to provide an electronic endoscope and an endoscope system having a configuration wherein with an electronic endoscope (any one of a rigid-type electronic endoscope and a flexible-type electronic endoscope) including an image capturing device at the distal end, and including a detachable mounting/detaching portion in the vicinity of the operating section, a surgeon can use the same functions and so forth as in the case of replacing the previously employed endoscope with another endoscope even during endoscopy.

In this case, another electronic endoscope means an electronic endoscope which is a new electronic endoscope subjected to sterilization, but has the same type as that prior to replacement, or an electronic endoscope having a different type from that prior to replacement (e.g., line of sight, thickness, length, functions, or the like differs).

The present embodiment employs a configuration wherein a front-side endoscope portion (first unit) and a rear-side endoscope portion (second unit) are freely detachably mounted at the mounting/detaching portion provided in the vicinity of the operating section, and thus allows a surgeon to employ this as an electronic endoscope having the same functions and so forth as in the case of replacing the entirety with another endoscope, even during endoscopy, by replacing the front-side endoscope portion alone. Employing such a configuration enables time and effort at the time of replacement to be saved.

Also, the present embodiment secures compatibility so as to be employed even in a light source device and a signal processing device in a case of a conventional electronic endoscope in which the mounting/detaching portion is not detachable. Electronic endoscopes will be abbreviated as endoscopes below.

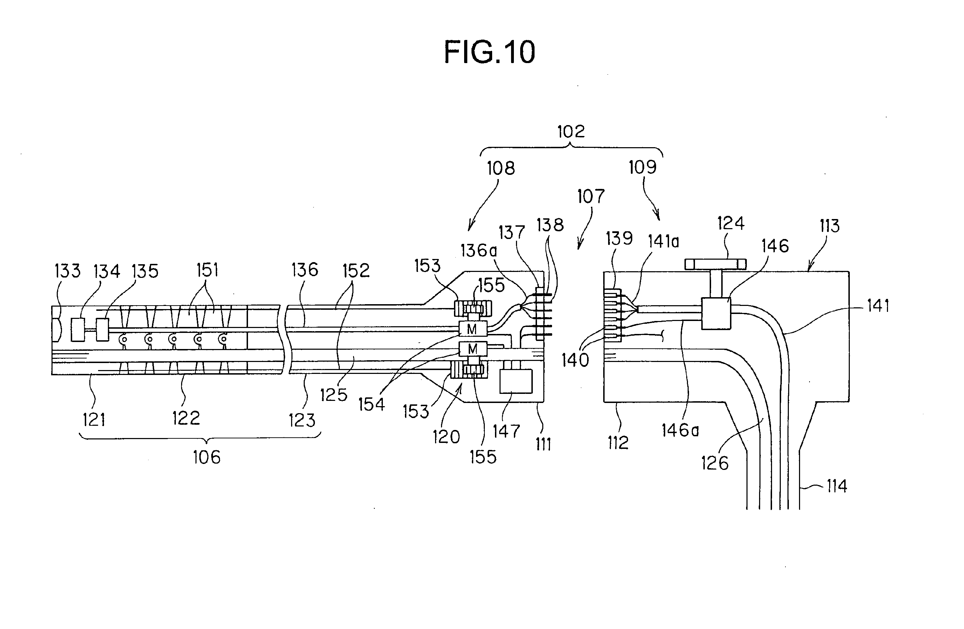

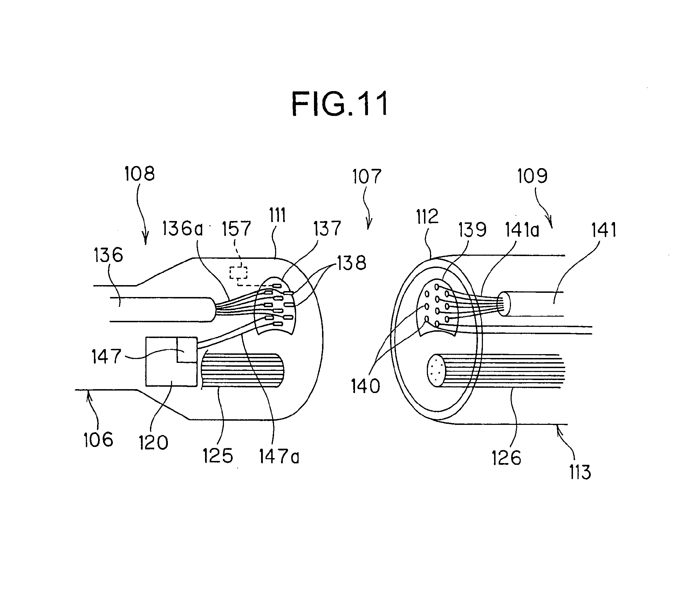

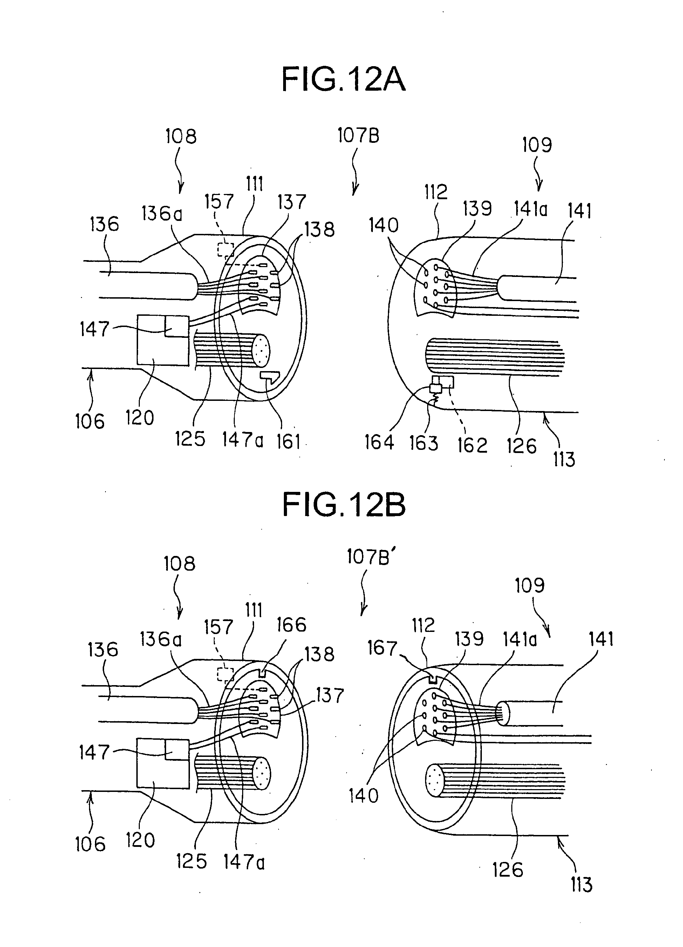

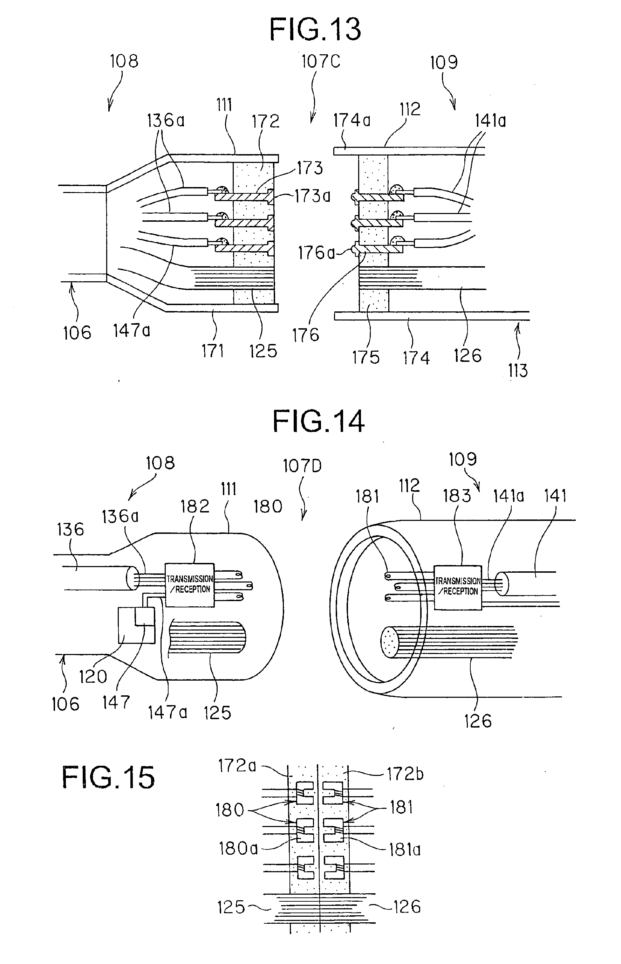

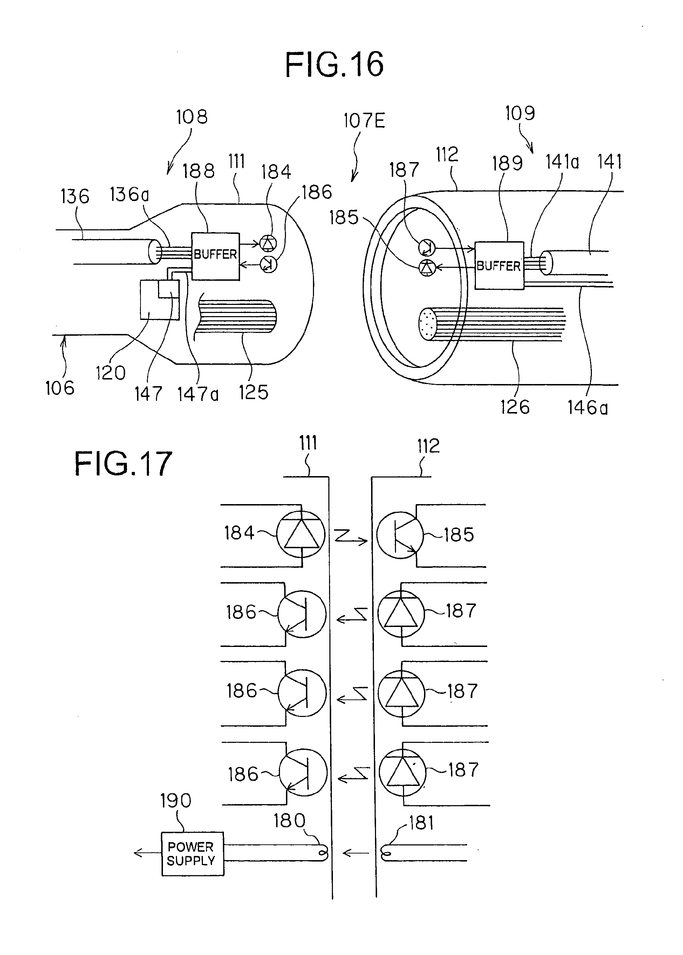

Fig. 9 illustrates an endoscope system including the fifth embodiment, Fig. 10 illustrates the configuration of the endoscope according to the present embodiment in which a mounting/detaching portion is provided, and Fig. 11 illustrates the configuration on the periphery of the mounting/detaching portion. Also, Fig. 12A and Fig. 12B illustrate the configuration of a mounting/detaching portion according to a first modification, Fig. 13 illustrates the configuration of a mounting/detaching portion according to a second modification, Fig. 14 illustrates the configuration of a mounting/detaching portion according to a third modification, Fig. 15 illustrates the configuration on the periphery of a coil in the mounting/detaching portion in a mounting state, Fig. 16 illustrates the configuration of a mounting/detaching portion according to a fourth modification, and Fig. 17 illustrates the configuration of a transmission/reception portion (optical transmission portion) using light.

An endoscope system 101 illustrated in Fig. 9 basically differs from the endoscope system 301 as a preceding example illustrated in Fig. 24 in that a configuration wherein an endoscope in which a mounting/detaching portion is provided is employed, which is the features in the present embodiment, and endoscopy can be readily performed as another endoscope by replacement.

The endoscope system 101 includes a first endoscope 102, a light source device 103 for supplying illumination light to the first endoscope 102, a processor device 104 for subjecting an image capturing device built in the first endoscope 102 to signal processing, and a monitor 105 for displaying an image captured by the image capturing device in response to a picture signal to be output from the processor device 104 being input.

As illustrated in Fig. 10, the first endoscope 102 is configured wherein both units of a front-side endoscope portion 108 serving as an insertion unit side and a rear-side endoscope portion 109 serving as an operating unit side (or cable side) are freely detachably connected (mounted) by one-touch operation at a mounting/detaching portion 107 in the vicinity of the proximal end of a flexible insertion section 106. In the following, the front-side endoscope portion 108 (serving as a first unit) is abbreviated as a front-side unit 108, and the rear-side endoscope portion 109 (serving as a second unit) is abbreviated as a rear-side unit 109.

The front-side unit 108 includes the flexible insertion section 106, and the proximal end of this insertion section 106 is provided with a connector 111 for connection.

The rear-side unit 109 includes an operating section 113 wherein a connector receptacle 112 to be freely detachably connected to the connector 111 is provided at the front end thereof, and a universal cable 114 extended from the operating section 113. The connector 115 at the proximal end of the universal cable 114 is connected to the light source device 103.

Also, an electric cable 117, which is connected to an electric connector 116 provided at the side face of the connector 115, is connected to the processor device 104 for performing signal processing. The picture signal generated by the processor device 104 is output to the monitor 105.

The insertion section 106 of the front-side unit 108 comprises a hard tip portion 121 provided at the distal end thereof, a bending portion 122 which can be bent, and a flexible portion 123 having flexibility. The bending portion 122 is configured so as to be bent via a bending driving portion 120 by performing an operation for turning a bending knob 124 provided at the operating section 113.

Also, a light guide 125 for transmitting illumination light is inserted into the insertion section 106, and the light guide 125 is optically connected to a light guide 126 within the rear-side unit 109 via the connector receptacle 112 to be connected to the connector 111. Further, the end of the light guide 126 is mounted to the light source device 103 via the inside of the universal cable 114 as a light guide base protruding from the end face of the connector 115.

A lamp 129 which is turned on by the lamp driving electric power from a lamp driving circuit 128 within the light source device 103 is passed through an aperture 130, and the illumination light condensed at a condenser lens 131 is cast into the end face of the light guide base.

The illumination light cast into the end face of the light guide base is transmitted by the light guides 126 and 125, and is emitted from the distal end face of the light guide 125 fixed to an illumination window of the tip portion 121, and illuminates a subject such as an affected portion or the like.

With the illuminated subject, the optical image of the subject is formed at the image-forming position by an objective lens 133 attached to an observation window adjacent to the illumination window. A charge-coupling device (abbreviated as CCD) 134 is disposed at this image-forming position.

This CCD 134 is connected to a signal cable 136 via a buffer IC 135 for performing current amplification, and the signal cable 136 is inserted into the insertion section 106, and reaches an electric connector 137 illustrated in Fig. 10 and Fig. 11 provided at the connector 111.

As illustrated in Fig. 11, with the electric connector 137, multiple contact pins 138 protrude, and the respective signal lines 136a of the signal cable 136 are connected to the proximal end of the contact pins 138.

Also, an electric connector receptacle 139 is also provided in the connector receptacle 112 facing the electric connector 137 provided in the connector 111. The sig nal lines in a signal cable 141 inserted into the operating section 113 side are each connected to the proximal end of a contact pin receptacle 140 provided at the electric connector receptacle 139.

An arrangement is made wherein inserting the respective contact pins 138 of the electric connector 137 into the respective contact pin receptacles 140 of the electric connector receptacle 139 enables the signal cables 136 and 141 to be electrically connected so as to perform mutual transmission of electric signals, and also enables mechanical connection (mounting) between the connector 111 and the connector receptacle 112 to be performed.

The signal cable 141 inserted into the operating section 113 is further inserted into the universal cable 114, and is connected to a CCD driving circuit 143 and a CDS circuit 144 within the processor device 104 via the electric connector 116 in the connector 115 and further the electric cable 117.

The optical image formed at the image capturing surface of the CCD 134 is subjected to photoelectric conversion by the CCD driving signal from the CCD driving circuit 143 by being applied to the CCD 134, and is output as a CCD output signal.

The CCD output signal is input to the CDS circuit 144, the signal components thereof are extracted to be converted into a base band signal, and this base band signal is input to a picture process circuit 145, and is converted into a picture signal to be output to the monitor 105, and the subject image captured by the CCD 134 is displayed on the display screen of the monitor 105 as an endoscope image.

Also, the picture signal to be output from the picture process circuit 145 is input to an aperture control circuit 132 within the light source device 103, a light adjust ment signal is generated by the aperture control circuit 132, the opening amount of an aperture 130 is controlled with this light adjustment signal to perform light adjustment so as to obtain a suitable amount of illumination light.

Also, as illustrated in Fig. 10, a potentiometer 146 is provided at the proximal end of the bending knob 124, and the potentiometer 146 is also connected to the contact pin receptacle 140 of the electric connector receptacle 139 via a signal line 146a.

The contact pin receptacles 140 are connected to a driving control circuit 147 constituting the bending driving portion 120 via the contact pins 138 to be connected thereto, and signal lines 147a to be connected to the contact pins 138.

The driving control circuit 147 electrically bends and drives the bending portion 122 based on the detection signal of the potentiometer 146 which generates the electric signal corresponding to the amount of operation of the bending knob 124. As illustrated in Fig. 10, the bending portion 122 is configured so as to freely rotatably connect to a plurality of bending pieces 151 using a rivet. Also, bending wires 152 are inserted into the insertion section 106 in the vertical and horizontal directions. With each of the bending wires 152, the tip thereof is fixed to the distal portion 121, and the rear end thereof is connected to a rack 153. Note that in Fig. 9 and Fig. 10, one pair of bending mechanisms as to the vertical or horizontal direction are illustrated. Actually, two pairs of bending mechanisms as to the horizontal and vertical directions are provided.

Each of the racks 153 meshes with a pinion 155 attached to the rotary shaft of a motor 154, and subjects the bending wire 152 to traction or relaxation by rotating and driving the motor 154, and in the event of subjecting the bend ing wire 152 to traction, the bending portion 122 is bent and driven in the direction thereof.

Each of the motors 154 is connected to the driving control circuit 147, and the driving control circuit 147 drives the bending portion 122 in response to the op eration of the bending knob 124 as described above.

Also, with the endoscope system 101 according to the present embodiment, as illustrated in Fig. 9, an arrangement can be made wherein, for example, a front -side unit 108B of which the type differs from the front-side unit 108 is connected to a rear-side unit 109, whereby endoscopy can be performed as a second endoscope.

The front-side unit 108B differs from the value of the diameter of the insertion section 6 in the case of the front-side unit 108 for example, and has an insertion section 106B of finer a diameter than that.

Also, the front-side unit 108B employs a CCD 134B of which the number of pixels is smaller than that of the CCD 134 in the case of the front-side unit 108, for example.

Also, the front-side unit 108B incorporates memory 157 which stores ID information in the vicinity of the connector 111, for example, for generating ID information unique to the front-side unit 108B. The memory 157 is connected to the contact pin 138 (to which the signal line of the signal cable 136 is not connected) in the electric connector 137 via a signal line.

In the event that the front-side unit 108B is employed as the second endoscope connected to the rear-side unit 109, the processor device 104 is configured so as to perform driving and signal processing corresponding to the CCD 134B serving as the second endoscope by reading out the ID information from the memory 157.

The other configurations are the same as those in the case of the front -side unit 108. Note that as illustrated with a dotted line in Fig. 11, an arrangement may be made wherein the front-side unit 108 also incorporates the memory 157 for generating ID codes. That is to say, the signal processing or the like corresponding to the CCD 134 built in the front-side unit 108 can be readily performed by storing the identification information unique to the front-side unit 108. Also, the processor device 104 incorporates a CPU 158 serving as control means, which controls operations of the CCD driving circuit 143, CDS circuit 144, and picture process circuit 145 depending on the ID information of the memory 157.

Thus, with the front-side unit 108 and the rear-side unit 109 of the present embodiment, electric signal transmission means using electric contacts and the light guides 125 and 126 are respectively formed so as to be faced at the mounting/detaching portion 107.

According to the present embodiment having such a configuration, forming the first endoscope 102 in which the front-side unit 108 and the rear-side unit 109 are connected (mounted) at the mounting/detaching portion 107 enables this endoscope to be used generally in the same way as the case of the endoscope 302 illustrated in Fig. 24.

That is to say, the first endoscope 102 can be used not only by connecting to the light source device 103 and the processor device 104 illustrated in Fig. 9, but also by connecting to the light source device 103 and the processor device 104 illustrated in Fig. 24. Also, the endoscope 302 in Fig. 24 can be us ed by connecting to the light source device 103 and the processor device 104 in Fig. 9. Accordingly, the endoscope 102 and the endoscope system 101 secure the compatibility as to the conventional endoscope 302 and light source device and so forth.

Also, with the present embodiment, during endoscopy using the first endoscope 102, in the event that the surgeon wants to continue the endoscopy by replacing the first endoscope 102 with an endoscope having a finer insertion section for example, the surgeon should remove the front-side unit 108 from the mounting/detaching portion 107, and connect the front-side unit 108B having the finer insertion section 106B to the rear-side unit 109.

Thus, the mounting/detaching portion 107 which is freely detachable by one-touch operation is provided in the vicinity of the operating section 113, so even during endoscopy, the surgeon can readily change the previously used endoscope as an endoscope having a different type by performing a simple process for replacing the front -side unit alone.

Also, following endoscopy as to a certain patient being completed, even in the event of performing endoscopy as to the next patient, the surgeon can set a state in which the next endoscopy can be performed by performing a simple process fo r replacing the front-side unit alone, whereby time and effort can be greatly saved.

Specifically, with upper gastrointestinal endoscopy for example, in order to make the transition to the next patient following endoscopy having been completed, the following processes are necessary in the event of the preceding example in Fig. 24.

That is to say, in addition to a process for replacing the endoscope 302 used at the time of endoscopy with another endoscope 302 subjected to sterilization, a process or the like for reconnecting the connector 309 portion to the light source device 303, and also at the same time, reconnecting one end connector of the electric cable 311 to be connected to the connector 309 to the processor device 304 is necessary, which requires time and effort.

On the other hand, according to the configuration of the present embodiment, the above object can be realized by performing a replacement process of only the front -side unit 108 portion from the mounting/detaching portion 107 in the endoscope 102, and accordingly, time and effort can be saved greatly.

Also, with the present embodiment, electric bending driving means are provided in the front-side unit 108, which can simplify the configuration of the mounting/detaching portion 107 which is freely detachable from the rear-side unit 109. Specifically, electric instruction means for instructing the bending direction and the amount of bending are provided in the rear-side unit 109, so the mounting/detaching portion 107 needs only a transmission unit for transmitting an electric signal, which can be shared with the transmission of another electric signal, thereby simplifying the configuration thereof.

Also, the bending knob 124 serving as electric instruction means generates the electric signals of the bending direction and the amount of bending, which needs sufficiently less amount of operating force as compared with the case of manually subjecting the bending wire 152 to traction, and accordingly, the present embodiment can improve operability in the case of a bending operation.

Also, the present embodiment employs a configuration of commonly using the rear-side unit 109, which can reduce economical burden of a user as compared with the case of preparing different endoscopes individually.

Fig. 12A illustrates the configuration of a mounting/detaching portion 107B according to a first modification. The mounting/detaching portion 107B differs from the mounting/detaching portion 107 in Fig. 10 in that a hook 161 and a recess 162 are each provided for positioning and the like in the circumferential direction in the case of connecting the connector 111 and the connector receptacle 112.

In order to improve positioning in the circumferential direction to one place in the circumferential direction of the connector 111, and a connection retention function at the time of connection, the hook 161 protruding substantially in an L-letter shape for example is provided at the connector 111 side, and the recess 162 into which this hook 161 is fitted is provided at the other connector receptacle 112 side.

An engagement protrusion 164 pressed by a spring 163 in the direction perpendicular to the direction where the hook 161 is inserted (upward direction in Fig. 12A) is provided in the vicinity of the entrance of this recess 162.

At the time of mounting, the user performs positioning of the hook 161 at the position of the recess 162, and operates the connector 111 in such a manner as pressing this against the connector receptacle 112.

In response to this operation, the taper portion of the tip of the hook 161 moves the protrusion 164 downwards so as to be fitted into the recess 162. Upon the hook 161 being mounted within the recess 162, the protrusion 164 returns upwards by the elastic force of the spring 163, and thus the hook 161 is prevented by a predetermined elastic force of the spring 163 from slipping from the recess 162.

The user can remove the connector 111 from the connector receptacle 112 by performing a detaching operation with greater force than this elastic force. The other configurations are the same as those in Fig. 10.

The present modification can be utilized for positioning in the case of connecting the hook 161 and the recess 162 at the mounting/detaching portion 107, and also can increase intensity for retaining connection in the case of connecting those. The present modification has the same other advantages as those in the case of the configuration in Fig. 11.

Note that in Fig. 12A, the hook 161 and the recess 162 are provided for positioning in the circumferential direction in the case of connecting the connector 111 and the connector receptacle 112, and retention of connection thereof, but such as a mounting/detaching portion 107B' illustrated in Fig. 12B, an arrangement may be made wherein scratch-shaped portions 166 and 167 are each provided, which are utilized for positioning in the case of connection so as to hook the scratch-shaped portions 166 and 167 mutually.

In other words, an arrangement may be made wherein in the event of conn ecting the connector 111 and the connector receptacle 112, unless positioning between the scratch-shaped portions 166 and 167 (in the circumferential direction) is performed, the connector 111 and the connector receptacle 112 cannot be connected.

The other configurations are the same as those in the case of Fig. 12A. Also, the advantages in the case of employing the configuration in Fig. 12B are generally the same as those in the case of Fig. 12A.

As for the other positioning means in the case of connecting the connector 111 and the connector receptacle 112, as illustrated in Fig. 12B for example, indicators 169a and 169b for positioning or the like may be provided on the outer circumferential surfaces or the like of the connector 111 and the connector receptacle 112.

Fig. 13 illustrates the configuration of a mounting/detaching portion 107C according to a second modification. With this mounting/detaching portion 107C, multiple contact pins 173 and the edge portion of the light guide 125 are firmly fixed so as to be embedded using a hard resin mold 172 for sealing the open end of a cylinder 171 serving as the external member of the connector 111.

The signal lines 136a and the signal line 147a to be connected to the driving control circuit 147 are connected by soldering to the proximal end of the respective contact pins 173 at the inside of the connector 111 which is sealed with the resin mold 172 having a function for sealing and also a function serving as an insulator. Also, electrodes 173a which are integrally provided at the tips of the respective contact pins 173 are provided on the outside surface of the resin mold 172 so as to form a flat face.

On the other hand, with the connector receptacle 112, the inside of the open end of a cylinder 174 serving as the exterior member of the connector receptacle 112 is sealed with an insulating plate 175 made up of rubber having elasticity for example, and also multiple contact pins (receptacles) 176 and the light guide 126 are firmly fixed with the insulating plate 175 so as to be embedded.

The signal lines 141a are each connected by soldering to the proximal end of the contact pins 176 at the inside of the connector receptacle 112 which is sealed with the insulating plate 175. Also, electrodes 176a which are integrally provided at the tips of the respective contact pins 176 are provided on the outside surface of the insulating plate 175 so as to slightly protrude from the end face of the connector receptacle 112.

The electrodes 173a and 176a come into contact with each other so as to provide an electrically conductive state by connecting the connector 111 and the connector receptacle 112 in a state such as pressing the respective end faces of the connector 111 and the connector receptacle 112.

Also, a fitting portion 174a for retaining a mounting state in which the inside diameter of the cylinder 174 is fitted into the outside diameter of the cylinder 171 of the connector 111.

The other configurations are the same as those in the fifth embodiment illustrated in Fig. 10 and so forth. With the present modification, the respective end faces of the connector 111 and the connector receptacle 112 are almost configured with a planar structure as illustrated in Fig. 13, and accordingly, removal of moisture and stains of the end faces in the case of performing cleansing and disinfection can be readily performed. The present modification has the other advantages generally the same as those in the fifth embodiment.

Fig. 14 illustrates the configuration of a mounting/detaching portion 107D according to a third modification. With the mounting/detaching portion 107C in Fig. 13, the contact pins 173 and the contact pins 176 are employed, but with these mounting/detaching portion 107D, coils 180 and coils 181 for performing non-contact signal transmission using electromagnetic coupling are employed.

A transmission/reception circuit 182 to which the signal lines 136a and 147a are connected, and multiple coils 180, which are connected to the transmission/reception circuit 182, for enabling bidirectional signal transmission using electromagnetic coupling are provided within the connector 111 of the front -side unit 108.

Also, a transmission/reception circuit 183 to which the signal lines 141a and 146a are connected, and multiple coils 181, which are connected to the transmission/reception circuit 183, for enabling non-contact bidirectional signal transmission using electromagnetic coupling are provided within the connector receptacle 112 of the rear-side unit 109.

The transmission/reception circuits 182 and 183 each amplify and supply a signal to transmit to the coils 180 or coils 181, and also amplify the signals transmitted by electromagnetic coupling between the coils 180 or coils 181 facing each other. That is to say, the transmission/reception circuits 182 and 183 have a function serving as a buffer for effectively transmitting a signal to be transmitted or received via the coils 180 and coils 181.

Also, the coils 180 and coils 181 are embedded within the resin molds 172a and 172b for example, and in the event of mounting the connector 111 and the connector receptacle 112, the coils 180 and coils 181 are provided so as to adjacently face each other, as illustrated in Fig. 15.

The respective coils 180 and coils 181 are formed by wrapping conductive wires around magnetic members 180a and 181a having, for example, a U-letter shape of which magnetic permeability is great respectively, which are configured so as to perform signal transmission effectively by using electromagnetic coupling between the coils 180 and coils 181 which face each other.

The other configurations are the same as those in the case of Fig. 13. As illustrated in Fig. 15, the present modification enables an electric signal to be transmitted or received in a state in which both end faces of the connectors 111 and 112 are planar faces, and also enables a configuration wherein both end faces are sealed with the resin molds 172a and 172b to be provided, whereby cleansing and disinfection can be readily performed in a short period of time.

In the event of the mounting/detaching portion 107 according to the fifth embodiment, the electric connector 137 and the electric connector receptacle 138 have a configuration wherein corrugated portions caused by ordinary electric contact points between the contact pins 138 and the contact pin receptacle 139 are included, so in the event of performing cleansing and disinfection or sterilization, cleansing and disinfection or sterilization is performed following the electric connector 137 and the electric connector receptacle 138 being worn with a waterproof cap to prevent a medical fluid, vapor, gas, and so forth from leaking out to the electric contact points.

Conversely, with the present modification, the mounting/detaching portion 107D having a contactless configuration not accompanying electric contact points using corrugated portions is employed, which facilitates a waterproof design, whereby cleansing and disinfection or sterilization can be performed without the above waterproof cap. That is to say, time and effort according to cleansing or sterilization process can be saved.

The present modification has the other advantages substantially the same as the case of the fifth embodiment.

Fig. 16 illustrates the configuration of a mounting/detaching portion 107E according to a fourth modification. With the present modification, an arrangement is made wherein transmission or reception of a signal in the mounting/detaching portion 107E is performed using a light signal between a light emitting element and a light receiving element in a non-contact manner (a later-described optical fiber may be employed).

The mounting/detaching portion 107E in Fig. 16 differs from the mounting/detaching 107D in Fig. 14 in that light-emitting diodes (abbreviated as LED) 184 and 185, and phototransistors (abbreviated as PT) 186 and 187 are employed instead of the coils 180 and coils 181. Also, buffer circuits 188 and 189 for amplification are employed instead of the transmission/reception circuits 182 and 183.

Fig. 17 illustrates the details of the transmission/reception portion using light in Fig. 16. For example, an LED 184 converts the CCD output signal subjected to current amplification from a buffer circuit 188 into a light signal, and outpu ts this to a facing PT 185 in a non-contact manner. The PT 185 converts the received light signal into an electric signal to output this to a buffer circuit 189, and the buffer circuit 189 amplifies the electric signal.

Also, each of multiple LEDs 187 co nverts the CCD driving signal into a light signal, and outputs this to the facing PT 186. The signal from the potentiometer 146 is also transmitted via the LED 187 and the PT 186.

Also, DC power required by the front-side unit 108 is generated by electromagnetic coupling using the coils 181 and coils 180.

The coil 181 of the rear-side unit 109 is driven by an AC signal, and the coil 180 of the front-side unit 108 facing this coil 181 is transmitted in a non-contact electromagnetic manner. This AC signal transmitted to the coil 180 is subjected to rectification and smoothing processing by a power supply circuit 190 to generate DC power. Subsequently, this DC power is supplied to elements and circuits which require DC power, such as the CCD 134 and so forth.

Note that DC power generating means are illustrated in Fig. 17, but this can be also applied to the case of the third modification in Fig. 14. The present modification has substantially the same advantages as those in the case of the third modification in Fig. 14.

### (Sixth Embodiment)

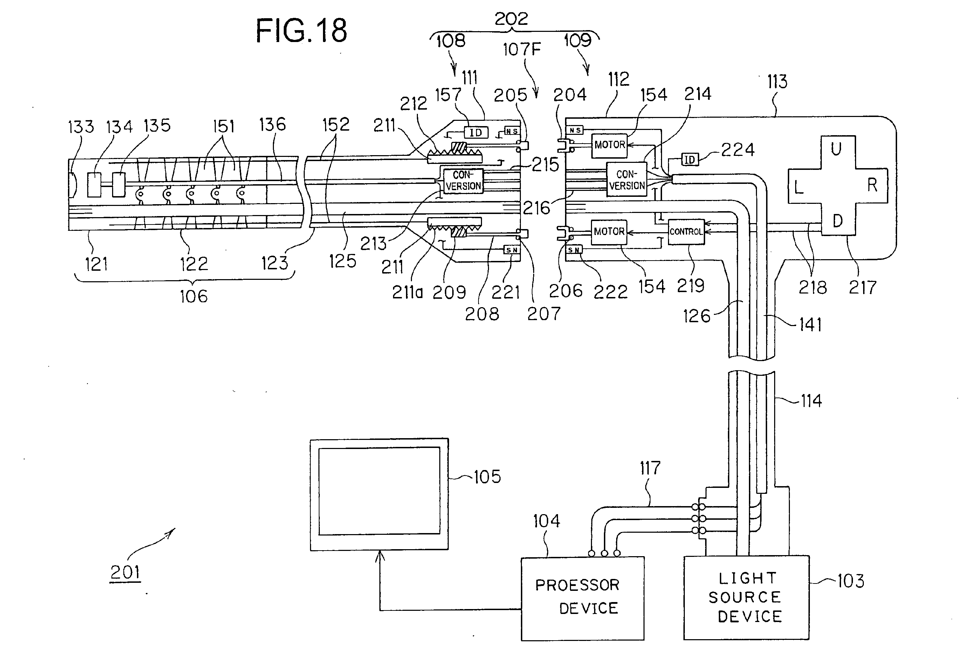

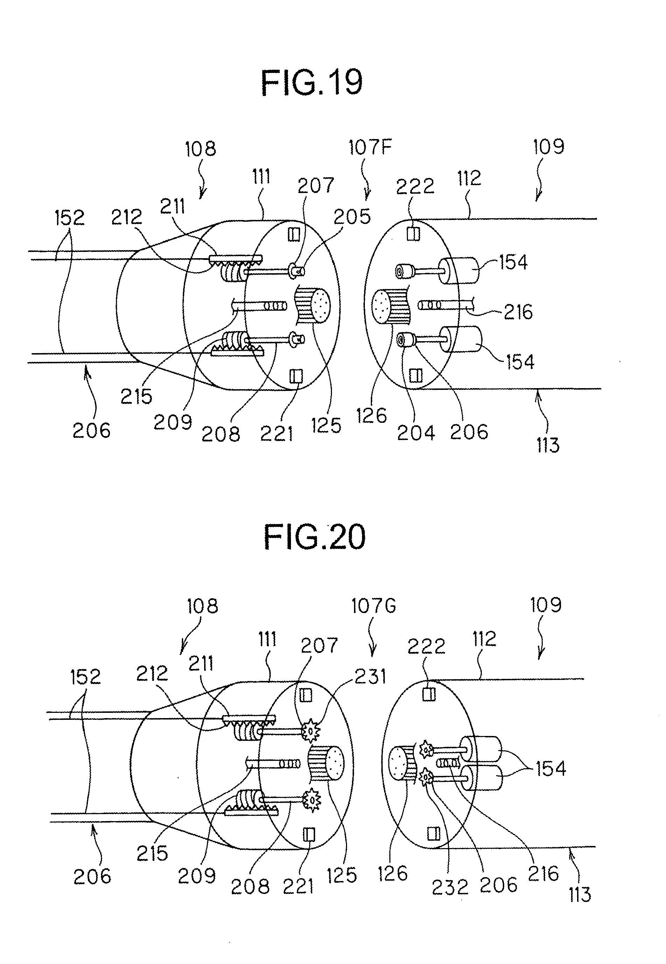

Next, a sixth embodiment of the present invention will be described with reference to Fig. 18 and Fig. 19. Fig. 18 illustrates an endoscope system 201 including the sixth embodiment.

The endoscope system 201 comprises an endoscope 202 according to the sixth embodiment, the light source device 103, processor device 104, and monitor 105 which are the same as those in the fifth embodiment.

With the endoscope 102 according to the fifth embodiment, the bending driving portion 120 serving as an electric bending driving means is provided at the front-side unit 108 side, but with the present endoscope 202, the motor 154 serving as a bending driving source is provided in the vicinity of the inside of the connector receptacle 112 for example within the operating section 113 of the rear-side unit 109.

The rotary shaft of the motor 15 4 transmits rotational power of the motor 154 by recesses 204, serving for engagement, protruding at the end face of the connector receptacle 112 being engaged with protrusions 205, serving for engagement, protruding from the end face of the connector 111 of the front-side unit 108. Note that O rings 206 and 207 for watertight and airtight applications are provided at the circumferences of the rotary shaft of the recesses 204 and protrusions 205 respectively.

A gear 209 is provided at the tip of a shaft member 208 where each protrusion 205 is provided, and each gear 209 is connected to the back end of the bending wire 152, and is geared with a corrugated portion 211a provided at a rod 211 which is movable.

An arrangement is made wherein rotation of the motor 154 moves the rod 211 forwards and backwards, and subjects the bending wire 152 to traction or relaxation, whereby the bending portion 122 can be bent.

Also, the signal cables 136 and 141 are each connected to conversion circuits 213 and 214, and also connected to optical fibers 215 and 216.

Conversion circuits 213 and 214 convert an electric signal into a light signal to output this to optical fibers 215 and 216, and also convert a light signal into an electric signal to output this to the signal cables 136 and 141, respectively.

For example, the CCD driving signal in the signal cable 141 is converted into a light signal by the conversion circuit 214, and is output to the proximal end face of the optical fiber 216, and this light is output to the facing optical fiber 215 side from the distal end face of the optical fiber 216.

Subsequently, the light signal in the optical fiber 215 is co nverted into an electric signal (CCD driving signal) by the conversion circuit 213, and is applied to the CCD 134 via the signal line of the signal cable 136. Inversely, the CCD output signal to be output from the CCD 134 side is converted into a light signal by the conversion circuit 213, and is input to the conversion circuit 214 via the optical fibers 215 and 216. Then, this light signal is converted into an electric signal (CCD output signal), and is input to the processor device 104 via the signal line of the signal cable 141.

Also, the outer circumferential surface of the operating section 113 is provided with a cross pad 217, serving for a bending operation, for example. With this cross pad 217, up, down, left, and right bending instruction switches are provided at four places shown with U, D, L, and R, and these switches are connected to a control circuit 219 provided within the operating section 113 via signal lines 218.

This control circuit 219 controls rotational driving of the motor 154 in ac cordance with a switch operation using the cross pad 217.

With the present embodiment, the end faces of the connector 111 and the connector receptacle 112 are provided with magnets 221 and 222 for positioning of mounting and retaining of a mounting state.

Also, these magnets 221 and 222 include a magnet portion and an electric contact portion, which are connected respectively.

That is to say, the magnet 222 connected to the power line of the signal cable 141 enables power to be supplied to the power line connected to the facing magnet 221. The power line connected to the magnet 221 becomes the signal line for power transmission of the signal cable 136.

Also, the memory 157, serving for generating ID information, is provided within the front-side unit 108, and this ID information is arranged so as to be transmitted to the rear-side unit 109 via the conversion circuit 213. Similarly, memory 224, serving for generating ID information, is also provided within the rear-side unit 109, and the information is arranged so as to be transmitted to the processor device 104.

The processor device 104 generates a CCD driving signal corresponding to the number of pixels, properties, and so forth based on the ID information, and also controls signal processing at the time of generating a picture signal. Note that only the one front-side unit 108 is illustrated in Fig. 18, but as illustrated in Fig. 9, another front -side unit having a different type or different properties or the like can be connected to the rear -side unit 109, and can be used.

Fig. 19 illustrates the configuration in the vicinity of the connector 111 and the connector receptacle 112 constituting a mounting/detaching portion 107F using a schematic view in a perspective manner.

With the present embodiment, electric bending driving means is provided at the rear-side unit 109 side, whereby the internal configuration of the front -side unit 108 can be simplified as compared with the case of the fifth embodiment.

That is to say, electric bending driving means is provided in the rear-side unit 109 which is commonly used, whereby the configuration of the front -side unit 108 serving as a replaced side can be simplified, and also costs can be reduced. The present embodiment has substantially the same other advantages as those in the fifth embodiment.

Fig. 20 illustrates the configuration of a mounting/detaching portion 107G according to a first modification. The present modification employs gears 231 and 232 instead of engagement using the corrugated portion according to the sixth embodiment. The other configurations are the same configurations as those in the sixth embodiment. The present modification has substantially the same advantages as those in the sixth embodiment.

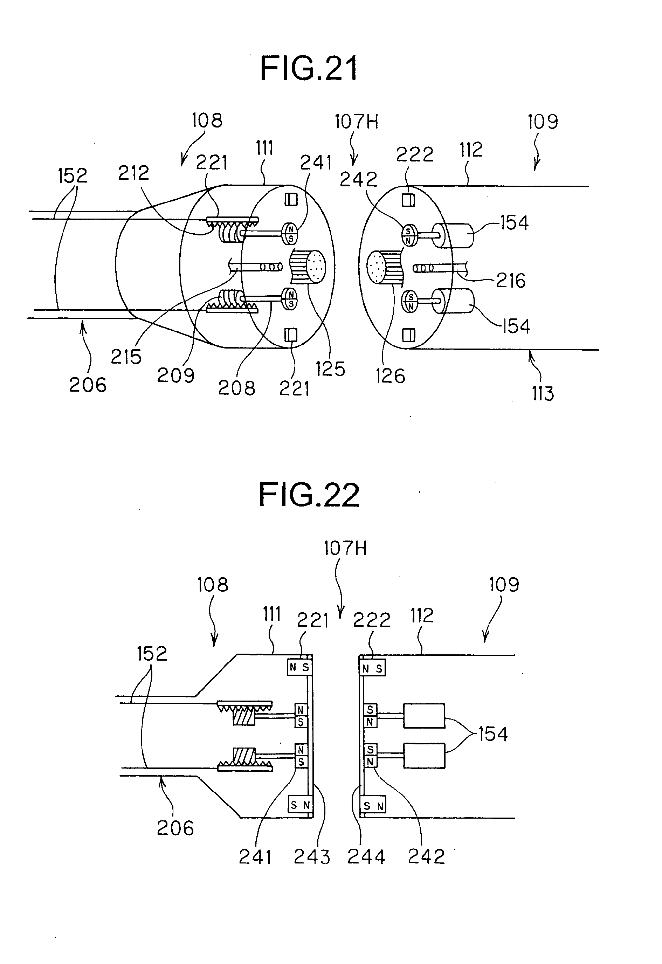

Fig. 21 illustrates the configuration of a mounting/detaching portion 107H according to a second modification. The present modification employs magnets 241 and 242 instead of engagement using the corrugated portion according to the sixth embodiment.

Specifically, in addition to rotation using the motor 154, the magnet 242 is rotated, whereby the magnet 241 disposed facing the magnet 242 which is affected by magnetic attraction can be also rotated.

Fig. 22 illustrates a schematic view in the case of viewing the configuration of Fig. 21 from the lateral direction. This case employs a configuration wherein the magnets 241 and 242 are disposed freely rotably at the inside of the seal plates 243 and 244 for sealing the respective end faces of the connector 111 and the connector receptacle 112.

Accordingly, the end faces of the connector 111 and the connector receptacle 112 can be made into planar faces, whereby cleansing and disinfection can be readily performed in a short period of time.

The other configurations are the same as those in the sixth embodiment. The present modification has an advantage wherein cleansing and disinfection can be readily performed, as described above. The present modification has the other advantages substantially the same other advantages as those in the sixth embodiment.

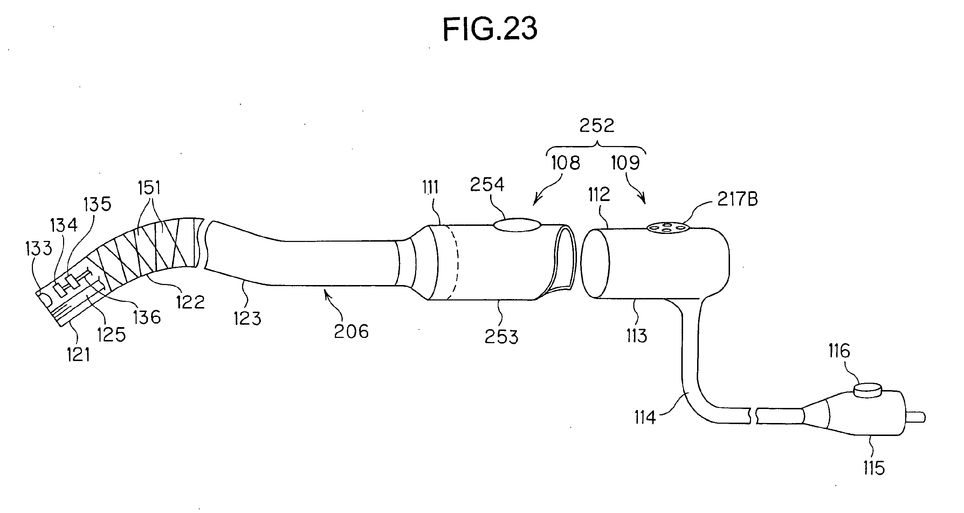

Fig. 23 illustrates an endoscope 252 according to a third modification. The endoscope 252 is an endoscope wherein an operating section cover 253 is provided in the front-side unit with the endoscope in Fig. 18, for example.

That is to say, an arrangement is made wherein with the outer circumferential surface of the connector 111, the operating section cover 253 extends at the backward side, whereby at least a front side portion of the operating section 113 of the rear-side unit 109 can be covered with the operating section cover 253.

Also, the operating section cover 253 is provided with a pad cover 254 which covers a substantially circular-disc-shaped bending operating pad 217B. The bending operating pad 217B is provided with switches which perform four-directional bending instructions, as with the case of the above cross pad 217.

The machinery and material portions which the surgeon touches during surgery, such as the operating section 113 and so forth, are subjected to sterilization processing before surgery beforehand, but as for another method, a method is available wherein a sack-like cover called a drape which has been subjected to sterilization processing is employed so as to cover the machinery and material portions instead of subjecting these portions to sterilization processing.

With the present modification, the operating section cover 253 serving as a flexible drape which is matched with a shape capable of covering the operating section 113 to be connected with the front-side unit 108 is integrally provided at the rear end of the front-side unit 108 which is often used.

Such a configuration enables another front-side unit 108 to be mounted and used without effort for replacing a drape at the operating section 113.

Note that embodiments made by combining parts or the like of the above embodiments or the like also belong to the present invention.

### Industrial Applicability

The present invention enables an endoscope to be separated into a front-side unit and a rear-side unit at a mounting/detaching portion provided in the vicinity of an operating section, whereby a surgeon can replace a previously used front-side unit with another front-side unit to readily perform endoscopy, even during endoscopy.

## Claims

1. An endoscope comprising:
a slender insertion section which incorporates image capturing means at the distal portion;
an operating section, which is provided at the rear end of the insertion section, to be gripped; and
a cable section in which a light guide cable for transmitting illumination light from the operating section and a signal cable connected to the image capturing means are extended integrally or separately;
wherein the connector of the end face of the cable section can be freely detachably mounted to each of a light source device and a signal processing device;
and wherein a mounting/detaching portion is provided between the front end and the back end of the operating section for freely detachably connecting a first unit serving as the insertion section side and a second unit serving as the cable section side.

2. The endoscope according to Claim 1, wherein the first unit and the second unit perform transmission of an electric signal between the first unit and the second unit by using an electric connector having an electric contact point to be freely detachably connected at the mounting/detaching portion.

3. The endoscope according to Claim 1, wherein the first unit and the second unit include optical transmission means for performing transmission of a signal between the first unit and the second unit by using optical coupling at the mounting/detaching portion.

4. The endoscope according to Claim 1, wherein the first unit and the second unit include electromagnetic transmission means for performing transmission of a signal between the first unit and the second unit by using electromagnetic coupling at the mounting/detaching portion.

5. The endoscope according to Claim 3, wherein the first unit and the second unit include power transmission means for performing transmission of power source by using electromagnetic coupling at the mounting/detaching portion.

6. The endoscope according to Claim 1, wherein the insertion section includes a bending portion which can be bent, and the first unit includes bending drivi ng means for electrically bending and driving the bending portion.

7. The endoscope according to Claim 1, wherein the insertion section includes a bending portion which can be bent, and the second unit includes bending driving means for electrically bending and driving the bending portion.

8. The endoscope according to Claim 7, wherein the first unit and the second unit include rotational power transmission means for transmitting rotational power by using the bending driving means at the mounting/detaching portion.

9. The endoscope according to Claim 8, wherein the rotational power transmission means includes engagement means constituted of a recess and a protrusion at the mounting/detaching portion.

10. The endoscope according to Claim 8, wherein the rotational power transmission means includes engagement means constituted of gears at the mounting/detaching portion.

11. The endoscope according to Claim 8, wherein the rotational power transmission means includes magnetic engagement means constituted of magnets at the mounting/detaching portion.

12. The endoscope according to Claim 1, wherein the first unit includes identification information generating means for generating identification information unique to the first unit.

13. The endoscope according to Claim 1, wherein with the second unit, a third unit having the same type as the first unit or a different type is freely detachably connected to the mounting/detaching portion.

14. The endoscope according to Claim 1, wherein the second unit includes bending instruction operating means for performing an instruction operation in a bending direction.

15. The endoscope according to Claim 14, wherein the bending instruction operating means is electric bending instruction operating means.

16. The endoscope according to Claim 1, wherein the first unit includes a cover for covering around the operating section of the second unit.

17. The endoscope according to Claim 1, wherein with the first unit and the second unit, illumination light transmission means for transmitting illumination light is provided at the mounting/detaching portion.

18. The endoscope according to Claim 3, wherein the optical transmission means comprises a light emitting element, and a light receiving ele ment.

19. The endoscope according to Claim 3, wherein the optical transmission means comprises optical fibers to be disposed facing each other.

20. An endoscope system comprising:
a slender insertion section which has image capturing means built in at the distal portion;
an operating section, which is provided at the rear end of the insertion section, to be gripped; and
a cable section in which a light guide cable for transmitting illumination light from the operating section and a signal cable connected to the image capturing means are extended integrally or separately;
wherein between around the front end and the back end of the operating section including
an endoscope including a mounting/detaching portion for freely detachably connecting a first unit serving as the insertion section side, and a second unit serving as the cable section side, and
a light source device and a signal processing device in which the connector of the distal portion of the cable section is freely detachably connected.

21. An endoscope, which has an insertion section and also incorporates image capturing means, comprising:
a light guide connection portion which is freely detachably connected with a light guide cable for transmitting illumination light from a light source device, which is inserted into the insertion section, and is provided at the incident end of a light guide for transmitting illumination light; and
a signal line connection portion which is freely detachably connected with a signal cable to be connected to a signal processing device for subjecting the image capturing means to signal processing, which is provided at the end face of a signal line electrically connected to the image capturing means.

22. An endoscope comprising:
an endoscope main body which has an insertion section and also incorporates image capturing means;
a light guide connection portion provided at the incident end of a light guide for transmitting illumination light inserted into the insertion section;
a signal line connection portion to which the end face of a signal line electrically connected to the image capturing means is connected; and
a cable unit, which is freely detachably connected to the light guide connection portion and signal line connection portion in the endoscope main body, into which a light guide for transmitting illumination light and a signal line for transmitting an electric signal are separately inserted, or at least a part thereof is commonly inserted.

23. The endoscope according to Claim 22, wherein the cable unit includes a connection portion to be integrally connected to each of the light guide connection portion and signal line connection portion.

24. The endoscope according to Claim 22, wherein the cable unit includes first and second connection portions to be freely detachably connected to each of the light guide connection portion and signal line connection portion.

25. The endoscope according to Claim 22, wherein the cable unit can be connected to each of the light guide connection portion and signal line connection portion via a connection adapter portion to be integrally connected thereto.

26. The endoscope according to Claim 24, wherein the cable unit includes third and fourth connection portions to be each freely detachably connected to a light source device and a signal processing device in addition to the first and second connection portions to be freely detachably connected to the endoscope.

27. The endoscope according to Claim 26, wherein the cable unit includes instruction means (switch means) for transmitting a control signal to the signal processing device.

28. The endoscope according to Claim 26, wherein the cable unit includes connection detecting means for detecting whether or not the first connection portion i s connected to the light guide connection portion.

29. The endoscope according to Claim 23, wherein the connection portion can be freely detachably mounted with an optical filter for restricting the wavelength band of illumination light to be incident into a light guide inserted into the insertion section.

30. The endoscope according to Claim 23, wherein the connection portion is provided with a light source portion for supplying illumination light to a light guide inserted into the insertion section.
